# EUROPEAN PATENT SPECIFICATION

(11) **EP 2 001 354 B1**
(45) Date of publication and mention of the grant of the patent: **13.01.2016**
(21) Application number: 07757854.0
(22) Date of filing: 02.03.2007
(51) Int. Cl.: A61B 5/0432, A61B 5/11

(54) **PHYSIOLOGIC MONITORING SYSTEMS AND METHODS**
PHYSIOLOGISCHE ÜBERWACHUNGSSYSTEME UND -VERFAHREN
SYSTEMES ET PROCEDES DE SURVEILLANCE PHYSIOLOGIQUE

(30) Priority: 03.03.2006 US 367992; 03.03.2006 US 367155; 03.03.2006 US 368290
(43) Date of publication of application: 17.12.2008
(60) Divisional application: 12171281.4
(73) Proprietor: Physiowave Inc., Palo Alto, California 94306 (US)
(72) Inventor: KOVACS, Gregory T.A., Palo Alto, California 94306 (US)
(74) Representative: Käck, Stefan
(86) International application number: PCT/US2007/063244
(87) International publication number: WO 2007/103835

(56) References cited:
- GB-A- 2 225 459
- US-A- 5 701 894
- US-A1- 2003 050 537
- US-A1- 2003 130 567
- US-A1- 2003 149 349
- US-A1- 2005 113 703
- US-B1- 6 594 759
- US-B2- 6 551 252

## Description

### BACKGROUND

The invention relates to physiologic monitoring systems and methods, and in particular to physiologic monitors.

Physiological monitoring instruments are used to measure a number of patient vital signs, including blood oxygen level, body temperature, respiration rate, and electrical activity for electrocardiogram (ECG) or electroencephalogram (EEG) measurements. In a common design used to perform ECG measurements, a number of electrocardiograph leads are connected to the patient's skin. Voltage variations are recorded over a period of time, and the resulting signals are processed, stored, and interpreted. The ECG signals of interest may be considerably lower in magnitude than environmental electrical noise levels generated by power lines, fluorescent lights, neighboring electrical devices, or electrolytic effects at the interface between the ECG leads and the patient's skin. The electrical signals sensed by the leads are commonly amplified and filtered in order to generate useful data.

A Holter monitor is an ambulatory electrocardiography device that allows heart monitoring for many hours or even days. Typical Holter monitors employ three to seven leads attached to a subject's skin. The monitor is commonly carried in a pocket or attached to a belt, and keeps a log of the heart's activity during a recording period.

A number of U.S. patents describe physiologic monitors, including portable ECG monitors. For example, in U.S. Patent No. 5,701,894, Cherry et al. describe an ambulatory physiological computer recorder that includes multiple selective plug-and-play signal input conditioners, a microprocessor system with operating and analyzing software, and a removable memory module for data storage. System sensors may include electrodes for ECG, as well as sensors for measuring body temperature, respiration, skin conductance, and acceleration, among others.

In U.S. Patent No. 6,198,394, Jacobsen et al. describe a system for remotely monitoring personnel status, including a plurality of sensors disposable on a soldier or other person for developing signals to determine the person's physiological status. Jacobsen et al. describe a wearable sensor unit including multiple sensors and a master controller or processor. Jacobsen et al. also describe employing a wrist sensor/display unit which may include multiple sensors and a controller connected to the sensors. The wrist unit is used in conjunction with a soldier unit carried by a soldier. The soldier unit also includes a controller, sensors, and other devices such as a global positioning system (GPS) device.

In U.S. Patent No. 6,454,708, Ferguson et al. describe a system for monitoring health parameters and capturing data from a subject. The system includes a cordless sensor band with sensors for measuring full waveform ECG, full waveform respiration, skin temperature, and motion, and a connector which accepts a memory card or a smart card for storage of measured data.

Further, U.S. Patent Application 2003/0050537 A1 discloses interactive reward devices and methods. According to the described solution, a patient is rewarded for performing a medical test or for maintaining medical test results within desired levels. Medical test data is generated and transferred for use by reward firmware in a cartridge. Reward information is provided to the patient to motivate or encourage the patient to conduct medical tests and/or maintain medical test results within certain levels. The cartridge can be inserted into an electronic controller, e.g. a handheld video-game controller, cellular telephones, or other devices. Transmission of data and/or encouragement to and from a remote location provides additional advantages.

U.K. Patent Application GB 2 225 459 A describes an event recorder which is designed to record only the data relevant to a specific event occurring. The event recorder comprises a non-volatile random access memory which is contained in a removable cartridge or credit card type of memory. Generated data is time-stamped and stored on the random access memory. The operating software is contained in a read only memory of the event recorder.

### SUMMARY

The invention is defined in claims 1 and 11, respectively. Particular embodiments of the invention are set out in the dependent claims.

According to one aspect, a physiologic monitoring system includes a physiologic monitoring application-specific integrated circuit (ASIC) including an integrated real-time clock for generating a set of real time indicators; integrated amplification and filtering circuitry for amplifying and filtering a set of electrical signals received from a first set of physiologic sensors to generate a first set of filtered electrical signals; and integrated digital control logic connected to the real-time clock and the amplification and filtering circuitry. The amplification and filtering circuitry includes analog filtering circuitry for filtering the set of electrical signals received from the first set of physiologic sensors. The digital control logic is configured to receive a first set of digital physiologic data derived from the first set of filtered electrical signals, receive a second set of digital physiologic data, wherein the second set of physiologic data includes physiologic data sampled with a different sampling rate than the first set of digital physiologic data, generate a time-stamped physiologic data packet from a real-time indicator, the first set of digital physiologic data and the second set of digital physiologic data; and transmit the data packet for storage in a digital memory.

According to another aspect, a physiologic monitoring integrated circuit includes mode-selection logic connected to digital control logic, for setting an operating mode of the integrated circuit to a mode selected from a stand-alone mode and a peripheral mode. In the stand-alone mode, the digital control logic is configured to generate a set of time-stamped physiologic data packets from the set of real-time indicators and a set of digital electrocardiogram data derived from filtered electrical signals, and transmit the time-stamped data packets for storage in the digital memory. In the peripheral mode, the integrated circuit is configured to transfer physiologic data derived from the digital electrocardiogram data to a programmable microcontroller.

According to another aspect, a physiologic monitoring method comprises storing a set of physiologic monitor configuration data in a removable digital memory; employing a wearable physiologic monitor including the digital memory to perform a set of physiologic measurements according to the configuration data on a subject wearing the monitor; and storing digital physiologic data generated by the physiologic measurements in the digital memory.

According to another aspect, a physiologic monitoring method comprises storing a set of physiologic monitor authorization data in a removable digital memory; transitioning a wearable physiologic monitor from an un-initialized state to an initialized state using the physiologic monitor authorization data stored in the removable digital memory, wherein the physiologic monitor in the un-initialized state is not user-operable to record a set of physiologic monitoring data from a subject, and wherein the physiologic monitor in the initialized state is user-operable to record the set of physiologic monitoring data from the subject; employing the physiologic monitor including the removable digital memory to record the set of physiologic monitoring data on the subject wearing the physiologic monitor; and storing the set of physiologic monitoring data in the removable digital memory.

According to another aspect, a physiologic monitoring method comprises: upon verifying that a subject's use of a wearable physiologic monitor is authorized, employing an initialization console to perform an activation of the physiologic monitor, wherein the activation transitions the physiologic monitor from a un-initialized state to an initialized state, wherein the physiologic monitor in the un-initialized state is not user-operable to perform a set of physiologic measurements, wherein the physiologic monitor in the initialized state is user-operable to perform the set of physiologic measurements; and using the physiologic monitor to perform a set of physiologic measurements on the subject and store digital physiologic data generated by the physiologic measurements in a digital memory of the physiologic monitor.

### BRIEF DESCRIPTION OF THE DRAWINGS

The foregoing aspects and advantages of the present invention will become better understood upon reading the following detailed description and upon reference to the drawings where:
Fig. **1-A** shows a schematic view of a wearable physiologic monitor placed on a subject.
Fig. **1-B** shows a more detailed view of the physiologic monitor of Fig. **1-A**.
Fig. **1-C** shows a number of components of the physiologic monitor of Fig. **1-B****.**
Fig. **2-A** is a diagram of the physiologic monitor of Figs. **1-A-C** in a stand-alone mode of operation.
Fig. **2-B** is a diagram of the physiologic monitor of Figs. **1-A-C** in a peripheral mode of operation.
Fig. **3-A** is a diagram of a signal drive and signal processing circuit of the physiologic monitor of Figs. **1-A-C.**
Fig. **3-B** is a diagram of a filtering circuit of the circuit of Fig. **3-A**.
Fig. **4** is a diagram of an exemplary digital control logic unit of the physiologic monitor of Figs. **1-A-C**.
Fig. **5** shows exemplary data packet contents.
Fig. **6-A** shows an exemplary initialization and analysis console and an authorization server for a physiologic.
Fig. **6-B** shows a set of software subsystems of the initialization console of Fig. **6-A**.
Fig. **7** shows a wearable physiologic monitor system including a wearable display connected to a wearable physiologic monitor.
Fig. **8** is a diagram of the physiologic monitor of Fig. **7**.
Fig. **9** shows an exemplary real-time display of physiologic data.

### DETAILED DESCRIPTION OF PREFERRED EMBODIMENTS

In the following description, it is understood that all recited connections between structures can be direct operative connections or indirect operative connections through intermediary structures. For example, digital control logic may be connected to amplification/filtering circuitry through an A/D converter. A set of elements includes one or more elements. A plurality of elements includes two or more elements. Any recitation of an element is understood to refer to at least one element. Unless otherwise specified, the term "logic" encompasses both dedicated (hardwired) logic and programmable logic such as logic implemented using field-programmable gate arrays (FPGA) or a programmable microcontroller. Unless otherwise specified, a wearable monitor encompasses monitors adhered to a subject (e.g. patches), as well as monitors loosely attached to a subject (e.g. through clothing, bands, string, a fanny pack, or other structures). Unless otherwise required, any described method steps need not be necessarily performed in a particular illustrated order. A first element (e.g. data) derived or generated from a second element encompasses a first element equal to the second element, as well as a first element generated by processing the second element and optionally other data. Generating a parameter of performing an action according to some data is not limited to generating the parameter or performing the action according only to that data, but encompasses using other data as well. An encapsulant is understood to be a generally-flexible material that encapsulates at least partially one or more enclosed components. Time-stamping first and second data encompasses applying a common time-stamp to a data packet including the first and second data, as well as separately time-stamping the first and second data with individual time-stamps. Time-stamping a data packet encompasses including an internal time-stamp within the packet, as well as associating an external time-stamp with the data packet. Unless otherwise specified, the statement that a digital memory is removable is understood to mean that the digital memory is removable by an end-user in the field by sliding or otherwise moving into and out of a mating position, without disassembling or destroying the memory or mating components. The statement that "amplification and filtering circuitry" includes a recited type of circuitry (e.g. continuous-time analog, switched capacitor, or DSP) means that at least some of the amplification and filtering circuitry includes the recited type of circuitry, and does not require both amplification circuitry and filtering circuitry to include the recited type of circuitry. Computer readable media encompass storage media such as magnetic, optic, and semiconductor media (e.g. hard drives, optical disks, flash memory, SRAM). The term "wide area network" encompasses the Internet as well as other networks including at least one router. Unless otherwise specified, the term "subject" encompasses both human and animal subjects. Unless otherwise specified, "home" use of a monitor refers to use during a normal course of activity of the subject and outside a medical setting (e.g. hospital or doctor's office), and is not limited to use at a home address of the subject. Replacing a first course of therapy with a second course of therapy encompasses employing a second course of therapy that is a modification of the first course of therapy (e.g. a modification in a medication dosage). Aspects of exemplary illustrated embodiments may contain patentable subject matter without regard to other aspects of the illustrated embodiments. Aspects of exemplary embodiments described below may be combined in ways other than the exemplary ways illustrated.

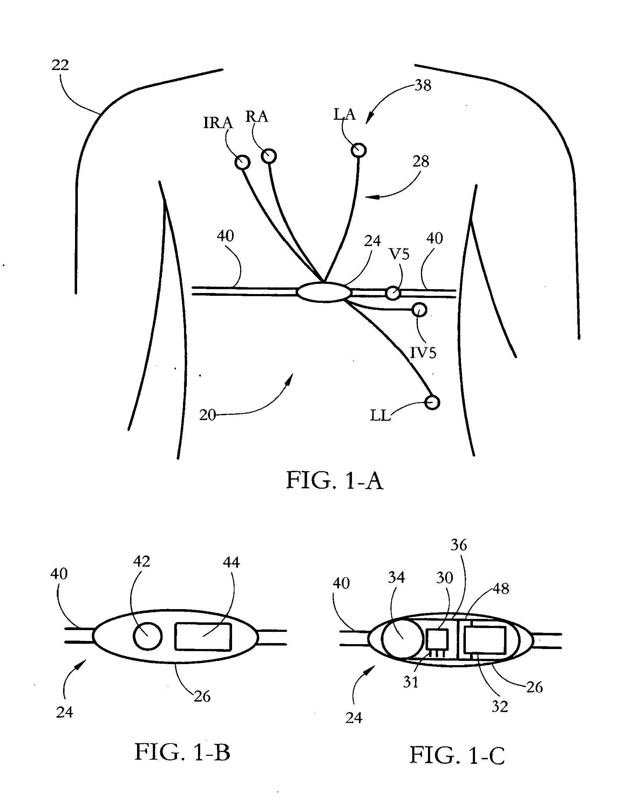

Fig. **1-A** shows a wearable physiologic monitoring system **20** placed on the skin of a subject **22,** according to some embodiments of the present invention. Subject **22** may be a human or animal subject. Physiologic monitoring system **20** includes a wearable physiologic monitor **24,** a plurality of subject electrical activity electrodes **38** attached to the subject's skin, and interconnections **28** connecting electrodes **38** to physiologic monitor **24**. In some embodiments, electrical activity electrodes **38** include electrocardiogram (ECG) electrodes, as well as respiratory and/or fluid load impedance electrodes. The exemplary six-electrode configuration shown in Fig. **1-A** includes LA (left arm), RA (right arm), IRA (respiratory/fluid load impedance RA), LL (left leg), V5 (left anterior axillary line in 5^{th} intercostal space), and IV5 (respiratory/fluid load impedance V5) electrodes. The LA, RA, LL and V5 leads are used for ECG measurements, while the IRA and IV5 leads are used for respiration and/or fluid load measurements. Other electrode configurations, including fewer or more electrodes and other electrode placements, are suitable for use in embodiments of the present invention. In some embodiments, electrodes **38** may include electroencephalogram (EEG) electrodes.

Interconnections **28** may include conductive wires with clips attached to electrodes **38**. Physiologic monitoring system **20** may include additional physiologic and external parameter sensors, as described in detail below. Electrodes **38** are electrically connected to physiologic monitor **24**. Physiologic monitor **24** may be formed by a patch held in place on the patient's skin by an underlying adhesive and/or a set of flexible straps **40**. In some embodiments, physiologic monitor **24** is a disposable bandage unit, which may include encapsulated electrodes and electrode interconnections. In some embodiments, physiologic monitor **24** may include a case attached to external electrodes through wires.

Fig. **1-B** shows a top view of physiologic monitor **24**. A sound and/or light indicator **42** and a user-activated event actuator (e.g. a button) **44** are situated on an outer surface of physiologic monitor **24**. In some embodiments, indicator **42** includes an LED providing visual system status indicators to a user. Indicator **42** may also include a speaker emitting status and/or prompt sounds (e.g. beeps, music, synthesized speech) for the user. Indicator **42** may include a D/A converter, low-pass filter, and power amplifier connected in sequence between its input and the speaker and/or LED. In some embodiments, light/sound indicator **42** may be replaced by or used in conjunction with an additional annunciator capable of providing vibratory or other tactile stimulation. Event actuator **44** allows a user to mark events of note (e.g. falling, feeling faint, unusual feelings, taking medications) by pressing actuator **44**. In some embodiments, user input actuators or controls may be provided in addition to actuator **44** to allow a user to enter a current date and time.

Fig. **1-C** shows several components held within an encapsulant **26** of physiologic monitor **24**. Encapsulant **26** may include a flexible material such as polyurethane. Encapsulant **26** partially encloses physiologic monitor **24**, to provide mechanical protection to physiologic monitor **24** while allowing external access to indicator **42**, event actuator **44**, and a battery **34**. Battery **34** may be sealed-in during a manufacture of physiologic monitor **24**, or inserted into physiologic monitor **24** by a user. A flexible substrate **36** supports battery **34** and an application-specific integrated circuit (ASIC) **30**. Integrated circuit **30** includes a set of input pins **31**, some of which may be bonded to a given logic level (0, 1) configuration to set an operating mode of integrated circuit **30**, as described below. Substrate **36** may include a non-conductive base and a set of conductive tracks (lines) formed on the base. In some embodiments, substrate **36** may be formed as described by Haines et al. in U.S. Patent No. 6,385,473, herein incorporated by reference.

A digital memory **32** is coupled to integrated circuit **30**, and stores processed physiologic data in digital form, as described below. In some embodiments, digital memory **32** includes a flash memory card removably mounted in a memory card connector (socket) **48** defined along substrate **36**. Suitable flash memory card formats include Compact Flash (CF) and xD-Picture card (xD), among others. In some embodiments, the digital memory may include a memory chip affixed to substrate **36**. In some embodiments, the digital memory may include on-chip memory integrated with ASIC **30**, as well as a non-removable, off-chip digital memory. In particular, in some embodiments, all data storage described below is performed in a removable memory card; in some embodiments, all data storage described below is performed in an on-chip memory; and in some embodiments, the bulk of measured physiologic data is stored in a removable memory card, while a set of configuration/initialization data is stored in an on-chip memory. Such configuration/initialization data may include settings and operational data such as firmware, subject ID, sensor sampling rates, authorization codes and data on authorized uses. An on-chip memory may also be used to provide temporary buffering of physiologic data while a memory card is exchanged.

Integrated circuit **30** is capable of operating in two or more alternative modes. Supported modes include a stand-alone mode, in which integrated circuit **30** stores data directly to digital memory **32**, and a peripheral mode, in which integrated circuit **30** is used as a peripheral to a microcontroller. In some embodiments, the mode of operation is set during a mounting and connection of integrated circuit **30** on substrate **36**, for example by bonding an input pin of integrated circuit **30** to one logic level (e.g. one, or V_{dd}) for one mode, and to another logic level (e.g. zero, or Vₛₛ) for the other mode. Integrated circuit **30** may be set to the stand-along mode if it is to be used in a disposable, wearable product, and to the peripheral mode if it is to be used in a reusable (wearable or bedside) product.

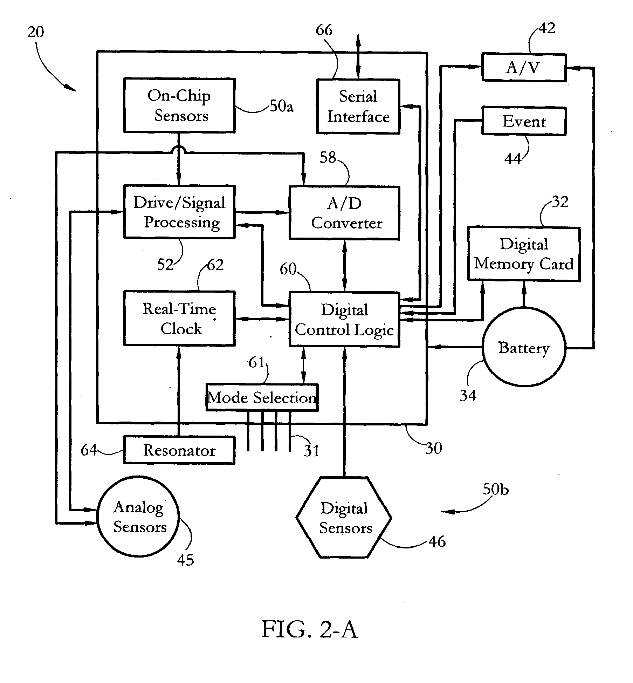

Fig. **2-A** shows a diagram of part of physiologic monitoring system **20** in a stand-alone mode of operation of integrated circuit **30**. Integrated circuit **30** includes a set of on-chip sensors **50a**, a signal drive and signal processing circuit **52**, an analog-to-digital (A/D) converter **58**, digital control logic **60**, mode-selection logic **61**, a real-time clock **62**, and an I/O port such as a serial interface **66**. Real-time clock **62** is connected to a resonator **64**. Resonator **64** may include an on-chip or off-chip timing resonator, and/or an off-chip quartz crystal.

A set of off-chip sensors **50b** includes a set of sensors **45** having analog outputs, and a set of sensors **46** having digital outputs. Analog sensors **45** may include the electrical activity electrodes **38** shown in Fig. **1-A**. In some embodiments, the set of on-chip sensors **50a** includes an integrated temperature sensor and integrated three-axis accelerometers, while other sensors are provided off-chip. In some embodiments, the set of on-chip sensors **50a** may include one or more of the sensor types described below. In some embodiments, the off-chip sensors **50b** include one or more accelerometers and/or tilt sensors, an electrical or mechanical respiration and/or fluid load sensor, an off-chip temperature sensor, a pulse oximeter, a light intensity sensor, an ionizing radiation sensor, a galvanic skin resistance sensor, a joint-angle goniometer, a strain sensor, and/or an acoustic sensor. Such sensors are configured to detect parameters within physiologically-relevant ranges known in the art.

The temperature sensor is thermally coupled to the subject's skin, and provides real-time temperature data indicative of the subject's current temperature. In some embodiments, an on-chip temperature sensor may include an on-chip diode. Measuring a diode's forward voltage employs a diode's natural temperature sensitivity to provide a temperature indication. An on-chip temperature sensor may also employ a temperature-dependence of a bandgap voltage to provide a temperature indication. For information on junction-based temperature sensors, which may be integrated in a CMOS or bipolar integrated circuit, see for example "Thermal Transducers," Chapter 6 of Kovacs, G. T. A., "Micromachined Transducers Sourcebook," McGraw-Hill, Inc., New York, NY, 1998, pp. 570-577. Preferably, the reading provided by the on-chip temperature sensor is substantially-independent of the power dissipation of integrated circuit **30**. Thus, preferably, the power dissipation of integrated circuit **30** is minimized, and the thermal coupling between the sensor and the subject is maximized, so as to minimize the effects of integrated circuit power dissipation on the temperature sensor output. In some embodiments, the temperature sensor is capable of measuring temperatures between 0 and 50 °C with an accuracy of 0.5 °C. In some embodiments, a temperature sensor may include an ambient temperature sensor, a skin probe contacting the subject for skin temperature measurements, or a rectal probe for core temperature measurements.

The accelerometers provide real-time data on the relative position of the wearer (e.g. standing, supine), as well as his/her dynamic movements (e.g. walking, running, sleep, etc.). One or more accelerators may be provided for each of the x-, y- and z-axes. In some embodiments, the accelerometers are DC-responsive accelerometers capable of measuring acceleration values up to a predetermined threshold, which may be chosen to be between 2 g and 5 g. DC-responsive accelerometers are capable of measuring both constant and time-varying accelerations. Constant accelerations are steady-state accelerations, such as the gravitational acceleration. Measurement of such steady-state accelerations may be used to determine subject body orientations, i.e. standing vs. lying down. Time-varying accelerations may include accelerations caused by walking. The set of accelerators may include accelerators capable of determining acceleration values, as well as activity sensors, or switch-type accelerators, which detect whether an acceleration threshold has been exceeded. A switch-type accelerator may include a movable mass which closes a pair of switch contacts when the accelerometer is subjected to a sufficiently high steady-state or dynamic acceleration. Multiple switch-type accelerometers with different thresholds may be used along an axis, in order to provide information regarding the magnitude of sensed accelerations.

An impedance-based respiration and fluid load/hydration sensor is capable of detecting a trans-thoracic impedance of the subject by applying an alternating-current (AC) signal to one electrode placed on the subject's skin, and detecting the subject's response to the applied signal through another electrode placed on the subject's skin. The electrodes may include the IRA and IV5 leads shown in Fig. **1**-**A**. The high-frequency part of the detected impedance is indicative of the subject's respiration, while the low-frequency or steady part of the impedance is indicative of the subject's fluid load/hydration. A mechanical respiration sensor may include a resistive band, applied to the subject's chest, having a resistance proportional to a subject chest's expansion. An exemplary respiration sensor may include a Procomp™ sensor, available from Thought Technology Ltd., West Chazy, NY.

A pulse oximeter is capable of detecting the subject's blood oxygen levels. A suitable pulse oximeter may include a Nonin® pulse oximeter (Nonin Medical Inc., Plymouth, MN), which may be attached to a finger, the forehead, or an ear lobe. Light intensity and acoustic sensors may be used to detect ambient light and sound levels, respectively. An ionizing radiation sensor may be used to detect levels of ionizing radiation such as gamma-radiation and X-ray energy. A galvanic skin resistance sensor may be used to measure the electrical resistance of the subject's skin, which depends on sweating, among other factors. A joint-angle goniometer may be used to measure the angle of a subject's joint. An exemplary joint-angle goniometer is available from ADInstruments, Inc., Colorado Springs, CO.

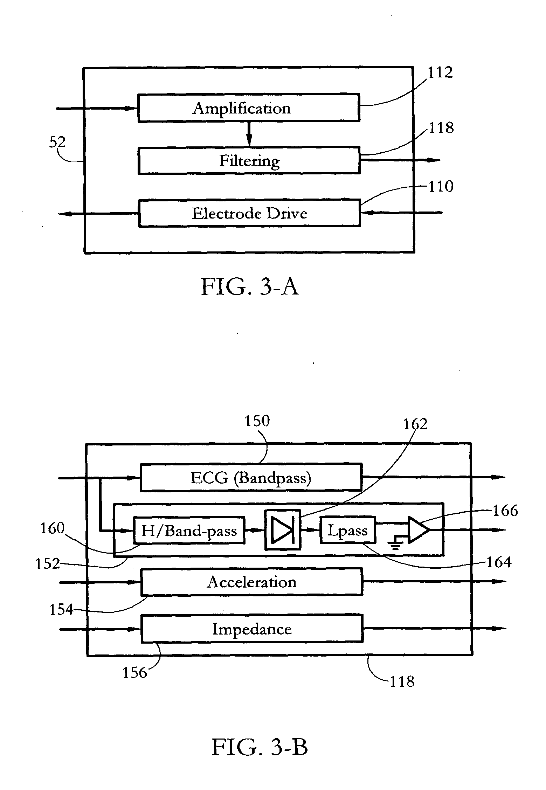

A signal drive and signal processing (signal conditioning) circuit **52** is connected to on-chip sensors **50a** and off-chip sensors **50b**, including analog sensors **45** and digital sensors **46**. Fig. **3-A** shows a diagram of drive/processing circuit **52.** In response to enable signals received from digital control logic **60**, a signal drive circuit **110** drives one or more analog sensors **45** (Fig. **2-A**). For example, if analog sensors **45** include impedance-based respiration measurement electrodes, signal-drive circuit **110** provides an AC drive signal (voltage or current) to one of the electrodes. In some embodiments, signal drive circuit **110** is used, under the control of digital control logic **60**, to identify inoperative ECG leads, such as leads that have may have become loose or been pulled off accidentally. Inoperative ECG leads are detected by an ECG fault detection circuit, which may include an AC signal (voltage or current) source, and a set of analog switches applying an AC test signal generated by the source to a selected ECG electrode being tested. An ECG lead fault is identified by measuring an impedance between the selected electrode and a reference (e.g. another electrode). An inappropriately high impedance value indicates that the ECG lead is disconnected or otherwise faulty.

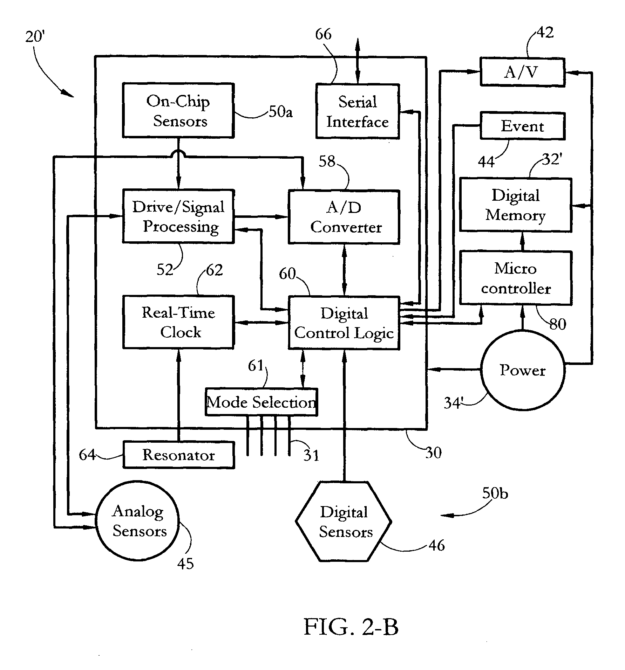

An analog amplification circuit (amplifiers) **112** receives sensed signals from on-chip sensors **50a** and off-chip analog sensors **45**, and amplifies the received signals. A filtering circuit **118** filters the signals, which are then transmitted to A/D converter **58** (Fig. **2-A**). In some embodiments, amplification circuit **112** and filtering circuit **118** may include multiple components used in parallel, for amplifying and filtering signals received from different types of sensors, which may correspond to different signal amplitudes, frequency content, and A/D sampling rates. Such components may include components used only in some configurations. For example, amplification circuit **112** and filtering circuit **118** may include circuitry capable of processing signals from twelve ECG leads and a number of sensor types described above, even though not all ECG leads and/or sensor types are used. A given sensor combination may be enabled by setting (e.g. bonding) a set of sensor configuration inputs (e.g. pins) of integrated circuit **30** to a combination of ones (e.g. V_{dd}, the circuit positive supply voltage) and zeros (e.g. Vₛₛ, ground or the circuit negative supply voltage). An exemplary sensor set configuration may use only two of twelve available ECG leads. In a peripheral mode (described below with reference to Fig. **2-B**), the sensor configuration mode of amplification circuit **112** and filtering circuit **118** may be set using a programmable internal register rather than bonded sensor configuration pins. In some embodiments, a given sensor combination may also be dynamically configured at a time of use, through digital control logic **60**.

Amplification circuit **112** may include one or more voltage and/or transresistance (transimpedance) amplifiers, depending on whether voltage or current is sensed. Amplification circuit **112** may also include one or more differential amplification circuits, particularly for ECG signal processing. Amplification circuit **112** amplifies received signals so that the amplitude of the signal output to AID converter **58** (Fig. **2-A**) corresponds generally to the full-scale input signal of A/D converter **58**. Generally, amplification circuit **112** is capable of amplifying received signals by a factor on the order of at least 10, e.g. by factors of hundreds or thousands. For example, if ECG signals received from electrodes **38** have a range on the order of ±5 mV, and A/D converter **58** has an input full scale on the order of 5 V, amplification circuit **112** may offset the input to 0-10 mV and amplify the resulting signal so that A/D converter **58** receives signals ranging from 0 to 5 V. Common CMOS A/D converters may have full-scale input voltages ranging from about V₅₅ (e.g. ground) to V_{dd} values such as 3.3 V or 5 V.

Filtering circuit **118** filters received signals, and transmits resulting signals to A/D converter **58**. Filtering operations performed by filtering circuit **118** include anti-aliasing, noise-rejection, and band-shaping. Generally, the properties of filtering circuit **118** may be chosen according to the signal frequencies and sampling rates of interest, which may depend on a set of corresponding enabled sensors. At least parts of filtering circuit **118** are implemented using continuous-time analog circuitry including capacitors and resistors. In some embodiments, at least parts of filtering circuit **118** may be implemented using discrete-time analog circuitry such as switched-capacitor circuitry. In some embodiments, at least parts of filtering circuit **118** may be implemented using a digital signal processor (DSP); in such embodiments, an A/D converter may be used to digitize analog physiologic signals before input to the DSP filter, and front-end filtering circuitry may be provided to process signals before transmission to the A/D converter. In some embodiments, different parts of filtering circuit **118** may be implemented using continuous-time analog circuitry, switched capacitor circuitry, and/or a DSP. For example, filtering circuit **118** may include a continuous-time analog anti-aliasing and noise-rejection filter stage followed by subsequent continuous-time analog, switched-capacitor, and/or DSP band-shaping filters.

Fig. **3-B** shows a diagram of filtering circuit **118**. In embodiments in which filtering circuit **118** includes a DSP, DSP parts of filtering circuit **118** may be downstream from A/D converter **58** (Fig. **2-A**). Filtering circuit **118** includes an ECG signal filtering circuit **150**, a pacemaker pulse detection circuit **152**, an acceleration filtering circuit **154**, and an impedance filtering circuit **156**.

ECG signal filtering circuit **150** includes one or more bandpass filters that filter received data to reduce out-of-band noise and prevent aliasing. ECG signal filtering circuit **150** transmits the resulting signals to A/D converter **58** (Fig. **2-A**). Common ECG filter cut-offs may be between 0.5 and 100 Hz. For a filter low-pass cut-off frequency of 100 Hz and a sampling rate of 256 samples/second (corresponding to a Nyquist frequency of 128 Hz), a filtering circuit with a roll-off of 72 dB between 100 Hz and 128 Hz may be used to attenuate undesired high-frequency signals below the dynamic range of a 12-bit A/D converter, with a 6 dB dynamic range per bit. In some embodiments, ECG filtering circuit **150** may include circuitry for carrying out inter-lead calculations such as deriving augmented ECG lead data by performing algebraic combinations using standard ECG lead data. For information on conventional frequency response specification data defined for ECG applications see for example the ANSI standard document "Ambulatory Electrocardiographs," ANSI/AAMI EC38-98. For descriptions of exemplary ECG filtering circuits see for example U.S. Patent Nos. 5,206,602 and 5,382,956.

Pacemaker pulse detection circuit **152** detects pacemaker pulses, and outputs a sequence of digital pulses each corresponding to a pacemaker pulse. Pacemaker circuit **152** may be connected to one or more of the ECG electrodes **38** (Fig. **1-A**). Pacemaker pulses are generally much narrower than normal ECG waveforms from the heart. In some embodiments, pacemaker pulse detection circuit **152** includes several circuits connected in series: a high-pass or band-pass filtering circuit (filter) **160**, a rectification circuit (rectifier) **162**, a low pass filtering circuit **164**, and a comparator circuit **166**.

Filtering circuit **160** may have a lower pass frequency on the order of kHz to tens of kHz (e.g. about 30 kHz), and a higher pass frequency on the order of tens to hundreds of kHz (e.g. about 100 kHz), to limit high-frequency noise. Rectification circuit **162** receives AC current and generates a rectified positive-voltage waveform. Low-pass filtering circuit **164** has a pass frequency on the order of Hz, for example about 5 Hz, chosen so as not to obscure or blur together individual pacing pulses. Low-pass filtering circuit **164** produces a pulsatile waveform, with each pulse corresponding to one pacemaker pulse signal. Comparator circuit **166** receives the pulsatile waveform, and outputs a digital output corresponding to each pacemaker pulse. Comparator circuit **166** may include a Schmitt trigger: In some embodiments, the output of low-pass filtering circuit **164** may be sent to A/D converter **58** (Fig. **2-A**), where the pacemaker pulse detection signal is sampled at a rate on the order of tens of Hz (e.g. 50 Hz). For a description of an exemplary pacemaker pulse detection circuit see for example U.S. Patent No. 5,448,997.

In some embodiments, acceleration filtering circuit **154** includes a low-pass filter. In some embodiments, impedance filtering circuit **156** includes a bandpass filter for reducing out-of-band-noise. The bandpass filter is followed by a homodyne or synchronous receiver, which mixes the signal received from the bandpass filter with a signal used by electrode drive circuit **110** (Fig. **3-A**) to drive electrodes **38**. The homodyne or synchronous receiver is followed by a low-pass filter. The output of the low-pass filter is a signal proportional to the detected impedance, which is rectified and transmitted to A/D converter **58**.

In some embodiments, amplification circuit **112** and filtering circuit **118** include circuitry for detecting ECG lead faults. A lead fault detection circuit may include an amplifier and a demodulator for measuring the impedance between a selected ECG lead and a reference, which may be another of the ECG electrodes, by measuring a level of a signal (voltage or current) detected in response to an application of an AC test signal using electrode drive circuit **110**. The fault detection circuit may also include a threshold detection circuit which determines when a selected electrode impedance is above a predetermined threshold. For a description of an exemplary ECG fault detection circuit see for example U.S. Patent No. 5,206,602.

A/D converter **58** (Fig. **2-A**) receives filtered signals from drive/signal processing circuit **52**, and generates corresponding digital signals for transmission to digital control logic **60**. In some embodiments, A/D converter **58** may be a 12-bit, multi-channel, low-frequency, low-power device, for example a successive-approximation device or a sigma-delta device. A/D converter **58** is capable of digitizing signals received from multiple sensors, which may be sampled at different rates. A/D converter **58** is chosen so it is capable of digitizing at the maximum aggregate data rate from all sensors. For example, in some embodiments, ECG data is sampled at 256 samples/second, respiration data at 64 samples/second, and acceleration at 16 samples/second, while subject temperature, SpO₂, and heart rate are sampled at 1 sample/second. AID converter **58** may include a multiplexer connected to multiple analog channels, for selecting a given data channel at a time for digitization. In some embodiments, A/D converter **58** may include a sample-and-hold circuit, which takes a snapshot of an analog signal and holds its value until its corresponding analog-to-digital conversion is completed.

Mode-selection logic **61** is connected to digital control logic **60** and input pins **31**. Mode-selection logic **61** sets an operating mode of digital control logic **60** according to a logic level configuration of a set of mode-selection input pins **31**. Available operating modes include a stand-alone mode, and passive and self-clocked peripheral modes, described in detail below.

Real-time clock **62** (Fig. **2-A**) generates real-time digital time signals, which are transmitted to digital control logic **60**. Real-time clock **62** may also generate date signals. An initial real time and date are set for real-time clock **62** during a system initialization, through digital control logic **60**. In some embodiments, real-time clock **62** remains accurate within 0.01 seconds to 1 second, for example about 0.1 seconds, over 24 hours. In some embodiments, real-time clock **62** may receive periodic base timing signals from the main integrated circuit synchronization clock that provides synchronization clock signals to digital control logic **60** and other components of integrated circuit **30**. In some embodiments, real-time clock **62** receives periodic base timing signals from timing resonator **64**. In some embodiments, timing resonator **64** may be an external quartz crystal such as a 32,768 Hz crystal. In some embodiments, timing resonator **64** may be an on-chip silicon resonator integrated within circuit **30**. For information on integrated MEMS silicon resonators see for example Nguyen et al., "An Integrated CMOS Micromechanical Resonator High-Q Oscillator," IEEE J. Solid State Circuits 34(4):440-455, April 1999, Nguyen, "Transceiver Front End Architectures using Vibrating Micromechanical Signal Processors," Dig. of Papers, Topical Meeting on Silicon Monolithic Integrated Circuits in RF Systems, Sept. 12-14 2001, p. 23-32, Nguyen et al., "Micromachined Devices for Wireless Communications," Proc. IEEE 86(8):1756-1768, Aug. 1998, and Nguyen, "Frequency-Selective MEMS for Miniaturized Low-Power Communication Devices, IEEE Trans. Microwave Theory Tech. 47(8):1486-1503, Aug. 1999.

Real-time clock **62** includes divider components (e.g. flip-flops, counters) for digitally dividing the received base timing signal to produce a real-time digital signal with a frequency of 1 Hz. For example, a 1-Hz real-time clock signal may be generated by dividing down a 2.097152 MHz integrated circuit synchronization clock signal by 2²¹ = 2.097152 x 10⁶, using twenty-one flip-flops connected in series. The 1 Hz tick signal is further input to one or more digital counters to generate a real-time digital time stamp transmitted to digital control logic **60**.

A 1 Hz tick signal has a frequency which is of the same order of magnitude as some physiological frequencies of interest processed by signal processing circuit **52**. Multiples of 1 Hz (e.g. 2, 4, 8 Hz, etc.) generated by a flip-flop divider chain may also be on the same order of magnitude as some physiological frequencies of interest. Preferably, signal processing circuit **52** is substantially unaffected by leakage or noise generated by real time clock **62**. In some embodiments, the effects of real-time clock **62** on signal processing circuit **52** are reduced by physically distancing real-time clock **62** and signal processing circuit **52** along the surface of integrated circuit **30,** for example by placing real-time clock and signal processing circuit **52** along opposite sides of integrated circuit **30**, or on opposite sides of other circuit units. Furthermore, in some embodiments a low-frequency real-time clock tick signal may include softened (e.g. obtuse, trapezoidal) edges, rather than square edges. A de-coupling off-chip capacitor, as well as separate power supplies, may be used in some embodiments to further isolate signal processing circuit **52** from real-time clock **62**.

Serial interface **66** is connected to digital control logic 60 and real-time clock **62**. Serial interface **66** allows connecting integrated circuit **30** bi-directionally to an external computer, to perform a number of initialization steps and/or to otherwise configure digital control logic **60**. In some embodiments, serial interface **66** may also be used to connect digital control logic **60** to an external microcontroller (e.g. microcontroller **80,** shown Fig. 2-**B**).

Sound/light indicator **42** and event actuator **44** are connected to digital control logic **60**. Digital control logic **60** controls the visual and/or acoustic output of unit **42,** and receives event signals from event actuator **44** for recording. Visual and/or acoustic indicators (e.g. LED blinking or changing color, speaker beeping, playing music or providing a spoken indication) are provided to indicate alterts or error signals, such as error signals indicating an improper positioning of electrodes, or warning signals indicating that monitored physiologic parameters are outside predetermined ranges. In some embodiments, sound/light indicator **42** comprises a speaker and a processing circuit connected to the speaker. The processing circuit may include a D/A converter, low-pass filter, and power amplifier connected in sequence between digital control logic **60** and the speaker.

Digital control logic **60** is further connected to digital memory **32.** In some embodiments, digital memory **32** may be provided on-chip, integrated within circuit **30.** In some embodiments, digital memory **32** comprises a non-volatile memory such as flash memory card, with a capacity sufficient to store the sensor data of interest over a period of about 24 hours or more. For example, a data rate of 100 samples per second at 2 bytes per sample corresponds to a daily storage requirement of about 17 MB. In such an application, a memory-having a capacity on the order of 32 to 64 MB would allow storing data over several days. Table 1 lists approximate storage requirements (in bytes) for several sampling rates and total storage periods, in a system using a 12-bit A/D converter and 2 bytes per sample.

**Table 1**

| Period | 24 hours | 48 hours | 7 days |
|---|---|---|---|
| Seconds/period | 86400 seconds | 172800 seconds | 604800 seconds |
| 100 samples/sec | 17.3 × 10⁶ bytes | 34.6 × 10⁶ bytes | 121.0 × 10⁵ bytes |
| 200 samples/sec | 34.6 × 10⁶ bytes | 69.1 × 10⁶ bytes | 241.9 × 10⁶ bytes |
| 500 samples/sec | 86.4 × 10⁶ bytes | 172.8 × 10⁶ bytes | 604.8 × 10⁶ bytes |
| 1000 samples/sec | 172.8 × 10⁶ bytes | 345.6 × 10⁶ bytes | 1.2 × 10⁹ bytes |

Battery **34** is connected to digital memory **32**, sound/light indicator **42** and integrated circuit **30**. In some embodiments, a DC voltage provided by battery **34** may be on the order of 0.5 V to 9 V. Battery **34** may be coupled to an on-chip charge pump (voltage converter) if an on-chip voltage higher than the voltage provided by battery **34** is needed. In some embodiments, multiple batteries may be connected to digital memory **32**, sound/light indicator **42**, and integrated circuit **30**.

Fig. **2-B** shows a diagram of part of a physiologic monitoring system **20'** including integrated circuit **30** set in a peripheral mode of operation. The peripheral mode of operation is particularly suited to bedside or other non-disposable device applications. In a peripheral mode of operation, digital control logic **60** is connected to a microcontroller **80**. In some embodiments, microcontroller **80** is soldered down on a board, and connected to integrated circuit **30** through bonded pins. In some embodiments, microcontroller **80** may be connected to integrated circuit **30** through serial interface **66**, through a special-purpose port of integrated circuit **30**, or through the memory card connector otherwise used to connect a removable memory card to integrated circuit **30**. Microcontroller **80** may be mounted in a socket defined along substrate **36** (Fig. **1-****C**). Microcontroller **80** is further connected to a power supply **34'** and to a digital memory **32'**. Digital memory **32'** may include a memory card, a hard disk drive, random access memory (RAM), and/or other types of digital memory. Power supply **34'** may include one or more batteries, as well as a power converter driven by power-line AC current.

In a peripheral mode, microcontroller **80** handles a number of functions otherwise performed by digital control logic **60**, described in detail below. In some embodiments, a peripheral mode may include two sub-modes: a passive peripheral mode, in which digital control logic **60** is substantially inactive and microcontroller **80** manages most of the low-level functions of integrated circuit **30**; and a self-clocked peripheral mode, in which digital control logic **60** performs a number of low-level functions described below, and generates hardware interrupts to microcontroller **80** in order to transfer assembled data packets to microcontroller **80** for further processing. The stand-alone (Fig. **2-A**) and peripheral (Fig. **2-****B**) modes may be better understood by considering an exemplary configuration and operation of digital control logic **60**.

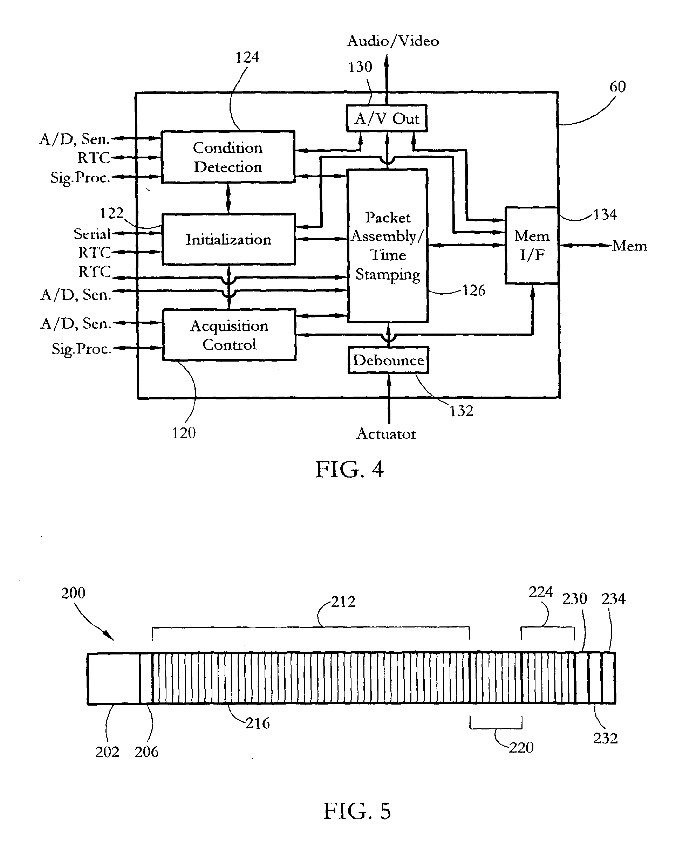

Fig. **4** shows a diagram of digital control logic **60.** Digital control logic **60** is a finite state machine (FSM). The diagram of Fig. **4** shows an exemplary configuration of functional blocks implementing a functionality of digital control logic **60**. The internal functional blocks of digital control logic **60** are shown in order to facilitate a systematic description of the functionality of digital control logic **60** according to some embodiments of the present invention, and not necessarily to imply required sharp structural boundaries within digital control logic **60**. An engineer may generate a hardware description language (e.g. Verilog, VHDL) description of digital control logic **60** that can be synthesized and implemented into a structure generated by electronic design automation (EDA) software. Such EDA software may partition the functionality of digital control logic **60** in other ways than the exemplary configuration of Fig. **4**.

The functional blocks of digital control logic **60** described below are connected and responsive to mode-selection logic **61** (Fig. **2-A**). The description below will focus on the operation of digital control logic **60** in a stand-alone mode. In a peripheral mode, mode-selection logic **61** disables/bypasses at least some of the functional blocks of digital control logic **60** described below. For clarity, the data acquisition description below focuses on data received from A/D converter **58** (Fig. **2-A**); additional data may be received from digital sensors **46** (Fig. **2-A**).

Digital control logic **60** includes acquisition control logic **120**, initialization logic **122**, condition detection logic **124**, packet assembly and time stamping logic **126** (for brevity, referred to below as packet assembly logic **126**), audio/video output control logic **130**, debounce and switch interface circuitry **132**, and a digital memory interface **134**. In some embodiments, digital control logic **60** is formed by hardwired logic. In some embodiments, digital control logic **60** may include a microcontroller core integrated on a common substrate with the other components of integrated circuit **30**, including the hardwired logic of drive/signal processing circuit **52** (Fig. **2-A**).

Digital memory interface **134** connects digital control logic **60** to digital memory **32** (Fig. **2-A**). In particular, digital memory interface **134** receives time-stamped physiologic data packets from packet assembly logic **126**, and directs the storage of the data packets in digital memory **32**. Digital memory interface **134** may add formatting/file system information to each packet before storage. The formatting information may depend on the file system used to store data in digital memory **32**. In some embodiments, a personal computer, DOS-compatible format such as a FAT16 or FAT32 format is used. Digital memory interface **134** selects a digital memory address for each packet to be stored. In some embodiments, selecting the address includes preventing over-writing data previously written to a removable memory by the present or another monitoring unit, such that the removable memory may be removed and re-inserted in and out of one or more monitoring units without causing substantial loss of data. Digital memory interface **134** determines the address of last-written data, and begins appending subsequent data following the last-written data, while maintaining a validly-formatted file. In some embodiments, digital memory interface **134** may support a single removable digital memory format. In some embodiments, digital memory interface **134** may support multiple removable digital memory formats, and may include pin-settable format selection logic for selecting a memory format to be used.

Initialization logic **122** is connected to serial interface **66** (Fig. **2-A**), real-time clock **62**, drive/signal processing circuit **52**, A/D converter **58**, and to a number of functional blocks of digital control logic **60** described below. In particular, initialization logic **122** is capable of connecting through serial interface **66** to an initialization console before physiologic monitoring system **20** becomes capable of acquiring and/or storing subject data.

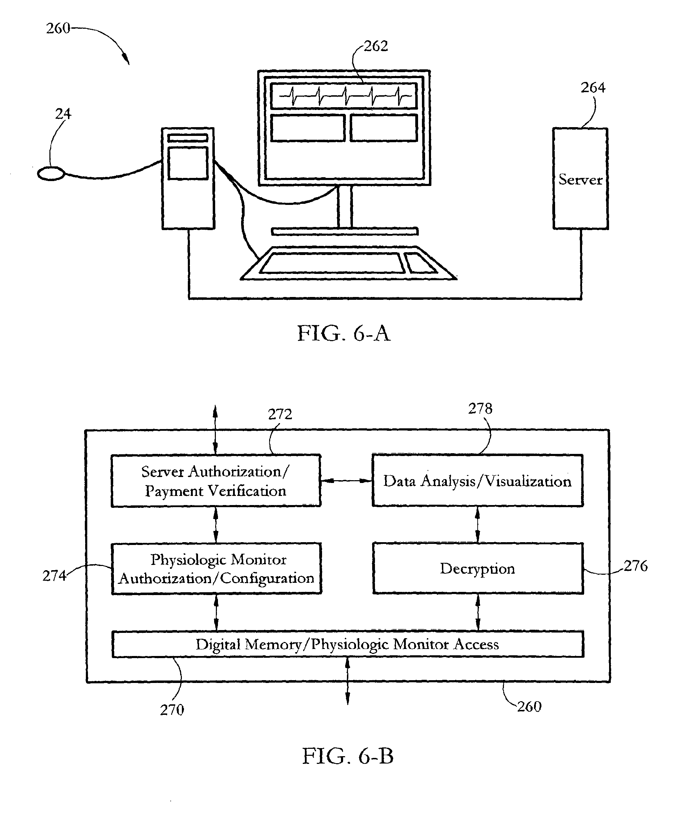

Fig. **6-A** shows an exemplary initialization and analysis console **260**, which may be implemented using software running on a general-purpose computer. In some embodiments, console **260** is connected to physiologic monitor **24** during an initialization of physiologic monitor **24**. In some embodiments, console **260** loads authorization data into digital memory **32** while the rest of physiologic monitor **24** is not connected to console **260,** and physiologic monitor **24** is subsequently initialized by retrieving the authorization data from digital memory **32.** In some embodiments, console **260** is also used to generate a physiologic data display **262** after a recording session has ended and data from digital memory **32** has been downloaded to console **260.** In some embodiments, console **260** is provided in a physician's office or other medical facility. Console **260** is also able to connect to an external authorization server **264.**

Fig. **6-B** shows an exemplary structure of initialization console **260**. A digital memory/physiologic monitor access unit **270** controls communications with physiologic monitor **24** and/or digital memory **32** to perform an initialization of physiologic monitor **24** and subsequent downloads of physiologic data. In some embodiments, the initialization and physiologic data download steps described below are performed while only digital.memory **32**, without the rest of physiologic monitor **24**, is connected to initialization console **260**. Access unit **270** may then include software and/or a removable digital memory reader allowing access to digital memory **32**. In some embodiments, the described initialization and/or download steps may be performed while integrated circuit **30** and/or microcontroller **80** (Figs. **2-A-B**) are connected to initialization console **260**. Access unit **270** may then include software and/or a connector for connecting to physiologic monitor **24**.

A server authorization and/or payment verification unit **272** connects initialization console **260** to external authorization server **264** (Fig. **6-A**) over a communications link such as a secure wide area network (e.g. Internet) or telephone connection. Payment verification unit **272** submits to authorization server **264** payment data (e.g. a credit or debit card number, or another payment indication), physiologic monitor identity data (e.g. one or more serial numbers uniquely identifying corresponding non-enabled physiologic monitors) and user identity data (e.g. a user's name) for a set of physiologic monitoring uses. In some embodiments, the payor may be the subject, while in others the payor may be a person or entity different from the subject. The set of paid uses may include the entire set of monitoring uses supported by physiologic monitor **24**, or a subset of the supported monitoring uses. Upon verifying that the user is authorized, for example if a payor has submitted a credit/debit card payment for a set of uses of physiologic monitoring system **20**, authorization server **264** transmits to initialization console **260** a set of authorization and/or other initialization data for physiologic monitor **24**.

In some embodiments, the authorization data includes a device-specific authorization code, as well as other subject- and session-specific authorization data. In some embodiments, a unique device-specific authorization code is required by physiologic monitor **24** in order to render physiologic monitor **24** user-operable to collect physiologic monitoring data, as described below. The authorization code identifies the stored data as originating from an authorized physiologic monitor. The authorization data may be traceable back to an individual physiologic monitor, subject, and payment authorization, in order to facilitate the creation of an audit trail. Initialization console **260** or an external server (e.g. authorization server **264**) may pool large amounts of subject data belonging to different subjects and sessions and identified by unique authorization data. In some embodiments, a correspondence between authorization data and the subject's identity (e.g. name) is maintained remotely on a secure central server, for example in a health care provider's office, in order to protect the subject's privacy and maintain confidentiality even if the data stored in digital memory **32** were accessed by an unauthorized person.

In some embodiments, the authorization data identifies a subset of authorized uses (e.g. enabled sensor subsets), which may be uses that the subject has paid for, and/or uses relevant to a medical condition of the subject. For example, ECG measurements may be enabled for heart patients with arrhythmias, fluid load impedance measurements for patients suffering from congestive heart failure, respiratory impedance measurements for patients for whom respiratory monitoring is desired, and accelerometers for patients whose calorie expenditures are to be monitored or who are at risk of syncope. A number of sensor combinations or subsets may be employed according to desired applications.

A physiologic monitor authorization/configuration unit **274** transmits a set of authorization and/or configuration data to digital memory **32.** The transmitted authorization and configuration data may include an authorization code, a physiologic monitor and/or subject ID, an encryption key identifier (an encryption key itself or other identifying data which may be used to retrieve or generate an encryption key), firmware, configuration settings for a number of components of physiologic monitor **24,** an initial real date and time, audio/visual prompts, reminders and alerts, and other data. The transmitted authorization and configuration data is described in detail below with reference to initialization logic **122** (Fig. **4**). In some embodiments, at least some of the transmitted authorization and/or configuration data may be received from authorization server **264**, and at least some may be generated by authorization/configuration unit **274**.

After physiologic monitor **24** has been used to record subject physiologic monitoring data for a period of time, digital memory **32** is re-connected to console **260** and the recorded data is downloaded to console **260**. A decryption unit **276** (Fig. **6-B**) receives from access unit **270** encrypted physiologic monitoring data recorded by physiologic monitor **24,** and decrypts the data using a decryption key associated with the recording session. The decrypted data is transmitted to a data analysis/visualization unit **278,** which generates physiologic data displays such as display **262** (Fig. **6-A**). In some embodiments, data analysis/visualization unit **278** verifies that received physiologic data was recorded using an authorized physiologic monitor prior to data analysis/visualization. The verification process may include confirming that an authorization code, physiologic monitor ID and/or subject ID are authorized. The verification process may include connecting to authorization server **264**.

On the physiologic monitor side, initialization logic **122** (Fig. **4**) receives an initialization command and/or initialization data from console **260** or digital memory 32, and directs an initialization sequence in response to the initialization command and/or data, prior to performing physiologic measurements. In some embodiments, to prevent piracy, digital control logic **60** is not user-operable to collect physiologic monitoring data prior to the initialization sequence. In some **embodiments,** initialization steps include conducting a self-test and calibration sequence, conducting a device authorization sequence, downloading firmware to configure microcontroller **80**, setting operating parameters to enable and/or configure signal processing circuit **52,** digital control logic **60**, and real-time clock **62**, and writing initial configuration data to digital memory **32** if needed.

In some embodiments, initialization logic **122** performs a diagnostic self-test sequence including checking a voltage of battery **34**, testing the ability of digital memory **32** to accept data (e.g. checking whether digital memory **32** is full and is working), checking sensor functioning by performing test measurements and/or fault detection steps for sensors **50a-b**, checking analog and/or digital circuitry functioning, and storing testing results within integrated circuit **30** or in digital memory **32**. Initialization logic **122** performs a calibration sequence including acquiring calibration data using sensors **50a**-**b**, and storing calibration factors in memory, within integrated circuit **30** or in digital memory **32**. The calibration factors include data generated from sensor measurements performed while physiologic monitoring system **20** is not connected to a subject.

In some embodiments, initialization logic **122** performs a device authorization sequence authorizing physiologic monitoring system **20** for a specific use. The device authorization sequence includes retrieving from initialization console **260** or digital memory **32** an authorization code and other authorization data to be associated with recorded physiologic data, and storing the authorization code and other authorization data in digital memory **32** and/or within digital control logic **60** if needed. As described above, in some embodiments initialization console **260** pre-loads digital memory **32** with authorization and/or configuration data, while digital memory **32** is removed from physiologic monitor **24**; in such embodiments, initialization logic **122** retrieves the authorization data from digital memory **32.** In some embodiments, the authorization data may be retrieved by initialization logic **122** directly from initialization console **260**.

In some embodiments, initialization logic **122** renders physiologic monitor **24** operable to record physiologic data only if a predetermined, physiologic-monitor-specific authorization code is received. In particular, in some embodiments, a part of a programmable logic array forming part of initialization logic **122** is programmed at manufacture to include authorization code detection logic which responds only to a device-specific authorization code, and permits activation of physiologic monitor **24** if the correct authorization code is received.

In some embodiments, the authorization data may include or otherwise identify an encryption key to be used by packet assembly logic **126** to encrypt physiologic data before storage in digital memory **32.** In some embodiments, the authorization data identifies a decryption key suitable for decrypting the physiologic data stored in digital memory **32.** Such a decryption key identification may be provided to physiologic data analysis software, together with physiologic data stored in digital memory **32,** for analysis after a recording session has ended.

In some embodiments, if a user has been determined to be authorized, initialization logic **122** retrieves from digital memory **32** or initialization console **260**: firmware for microcontroller **80** (Fig. **2**-**B**), configuration settings for digital control logic **60,** and filter coefficients, topologies and/or state machine instructions for filtering circuit **118** if filtering circuit **118** includes DSP circuitry. In particular, microcontroller **80,** digital control logic **60** and filtering circuit **118** may be operational only in a test mode or non-operational upon manufacture, and are only enabled to perform physiologic monitoring operations upon initialization. Digital control logic **60** may include a blank (re)programmable logic array that is only programmed during system initialization. For example, some logic functions of digital control logic **60** may be implemented using EEPROM or flash memory. In some embodiments, all of part of acquisition control logic **120**, condition detection logic **124,** and packet assembly/time stamping logic **126** (Fig. **4**) may include parts of (re)programmable logic array. The firmware download and digital control logic and filtering circuitry programming provide additional piracy protection, since microcontroller **80** and integrated circuit **30** become capable of acquiring physiologic data only upon authorization by the initialization console. Allowing programming of parts of digital control logic **60** also facilitates future design changes.

Initialization logic **122** receives an initial time/date setting from initialization console **260**, and uses the received initial time/date setting to set a current time/date for real-time clock **62** (Fig. **2-A**). Setting configuration parameters for signal processing circuit **52** and digital control logic **60** may include setting sampling rates and enabling operating features. Enabling operating features may include defining an enabled sensor set and/or sensing operations (e.g. impedance sensing operations) to be performed by signal processing circuit **52** and digital control logic **60**. An enabled sensor set may specify which sensor types or subsets (e.g. which ECG leads or other sensor types) are enabled for the authorized use. Different operating features and/or sampling rates may be enabled or set for different applications: for example, higher sampling rates may be used for monitoring the training of an elite athlete than for routine monitoring of an elderly patient or for veterinary uses. If filtering circuit **118** (Fig. **3-A**) includes digital signal processing (DSP) circuitry, setting configuration parameters for signal processing circuit **52** may include programming filter coefficients, topologies, and/or state machine instructions to customize and render operable signal processing circuit **52** upon initialization.

Initial configuration data written to digital memory **32** may include an authorization code, described above, as well as a physiologic monitor and/or subject ID. In some embodiments, initial configuration data written to digital memory **32** includes configuration parameters (e.g. enabled operating features, sampling rates) for signal processing circuit **52** and digital control logic **60**. Initial configuration data written to digital memory **32** may further include customized audio/visual alarms, prompts, and reminders to be played back to the user, as well as configuration settings for the stored audio/visual data. Audio prompts may include spoken reminders to take medicines, to remove monitoring unit **20** and/or digital memory **32** when digital memory **32** is full or when a programmed recording session is over, and indications of sensor faults or physiologic parameter conditions (e.g. a warning of a high measured heart rate, or praise for meeting a target exercise heart rate). In some embodiments, audio prompts are recorded using a voice such as the voice of the subject, a family member, a medical professional (e.g. the subject's family doctor or a familiar nurse), a public personality (e.g. an actor or professional athlete), a simulated famous person, or a cartoon character. In some embodiments, programmed speech data includes data personalized with both the user's identity, and with a number of speech messages tailored to a sensor subset or application. For example, if the user's name is Bob, and if Bob's physiologic monitor uses a set of ECG leads, programmed speech data may include a phrase such as "Bob, a lead has fallen off!" Audio prompts may also include music and/or sound effects. In some embodiments, configuration settings for the stored audio/visual data include audio volume configuration data causing the volume of audio output to be softer at predetermined times (e.g. during traditional sleep hours), or louder for patients who are hard of hearing.

Acquisition control logic **120** includes logic controlling the operation of A/D converter **58** and signal processing circuit **52** (Fig. **2-A**) according to a number of configuration parameters, including parameters defining the analog signals to be processed and their sampling rates. In embodiments in which signal processing circuit **52** uses analog circuitry, acquisition control logic **120** mainly handles the timing of A/D converter **58**. In particular, acquisition control logic **120** sends selection signals to a multiplexer of A/D converter **58**, to determine which of multiple analog signals is to be digitized. If A/D converter **58** includes a sample-and-hold circuit, acquisition control logic **120** sends hold pulses or sets hold logic levels to direct the sample-and-hold circuit to hold given analog signals. Acquisition control logic **120** further sends digitization commands to A/D converter **58,** directing A/D converter **58** to digitize data from a given signal channel. For example, if A/D converter **58** is a successive approximation device, acquisition control logic **120** may send a start digitization signal to A/D converter **58** to begin digitization of a sample. In some embodiments, a sample is considered ready following a predetermined number of synchronization clock cycles after its digitization start. In some embodiments, a sample is considered ready when a sample ready signal is received from A/D converter **58.**

In embodiments in which signal processing circuit **52** uses switched capacitor signal processing, acquisition control logic **120** may additionally provide a clock signal or signals to filter elements of signal processing circuit **52.** Hardwired logic within signal processing circuit **52** of acquisition control logic **120** may be used to provide desired switching frequencies to the filtering elements of signal processing circuit **52.**

In embodiments in which signal processing circuit **52** uses digital signal processing, acquisition control logic **120** may additionally be used to route a continuous sample stream to different filter channels, load and/or recirculate filter coefficients and resulting intermediate data, and carry out inter-lead calculations such as deriving augmented ECG lead signals by performing algebraic combinations of standard ECG lead signals.

Condition detection logic **124** receives signals from A/D converter **58,** signal processing circuit **52**, and real-time clock **62**, and detects whether predetermined physiologic conditions, real-time conditions, and/or sensor faults or other conditions have occurred. Condition detection logic **124** may also be used to control electrode drive circuit **110** (Fig. **3-**A) to apply AC fault detection signals to selected ECG electrodes. ECG lead fault detection steps may be performed after an initial placement of the ECG electrodes on the subject, and at periodic intervals thereafter. In some embodiments, condition detection logic **124** determines whether a measured impedance between a selected ECG lead and a reference (e.g. another ECG lead) exceeds a predetermined threshold. In some embodiments, condition detection logic **124** determines whether physiologic data received from A/D converter **58** (e.g. a measured subject temperature or heart rate, measured ionizing radiation levels, estimated calorie expenditures derived from accelerometer data) are outside predetermined ranges or otherwise meet predetermined conditions. In some embodiments, condition detection logic **124** determines whether a current real time and a measured ambient light level meet predetermined conditions (e.g. have predetermined values, or are lower or higher than predetermined values). Condition detection logic **124** sends condition indicators, which may include indicators of fault or other condition types, to audio/video output control logic **130** and to packet-assembly logic **126**. For example, when an estimated calorie expenditure during an exercise period has exceeded 300 calories, condition detection logic **124** may send a corresponding condition indicator to output control logic **130** in order to provide encouragement to the subject. When a real time is sufficiently late and/or a detected ambient light level is sufficiently low, condition detection logic **124** may send a quiet-volume indicator to output control logic **130**, in order to lower the volume or mute audio indicators. For ECG data, a sensor fault may include an indicator that one or more ECG leads have fallen off or are not connected properly. The fault indicators may be used by audio/video output control logic **130** to generate audio and/or visual warnings to a user. The condition indicators may be used by packet assembly logic **126** to include condition-detection flags in corresponding data packets, as described below.

Packet-assembly logic **126** receives data streams corresponding to different physiologic data types from A/D converter **58** and digital sensors **46**, and real-time indicators from real-time clock **62** (Fig. **2-A**). The data streams are received at different rates. Packet-assembly logic **126** assembles the received data into formatted packets for transmission to memory interface **134**, to be stored into digital memory **32** (Fig. **2-A**). Packet-assembly logic **126** includes one or more buffers for storing assembled data before transmission to memory interface **134.** Each packet sent to memory interface **134** includes a set of physiologic data of interest, a set of data types, associated time stamps, and a fault detection flag.

In some embodiments, packets are stored in digital memory **32** at regular intervals, for example one packet every second. A packet may include physiologic data of different types, sampled at different rates. For example, an exemplary packet stored every second may include 512 ECG samples (256 samples per second for 2 leads), 64 respiratory impedance samples, 48 acceleration samples (16 each of 3 axes), and other samples such as one or two SpO₂ samples, an event marker, and light, acoustic, ionizing radiation, and joint-angle goniometer samples. The ECG samples may be 12-bit samples, while other samples may be 8-bit samples. Together with time stamp and formatting data, such an exemplary packet may include on the order of a thousand bytes.

Fig. **5** shows the contents of an exemplary physiologic data packet **200** according to some embodiments of the present invention. Packet **200** includes a formatting field **202**, a time-stamp field **206**, an ECG data field **212** including multiple ECG data samples **216**, other physiologic data fields **220, 224, 230, 232**, and an event marking and fault detection field **234**.

In some embodiments, each stored packet is time-stamped by including a real-time indicator in the packet. In some embodiments, at least some of the packets need not include stored time-stamps; rather, the time of each packet can be extracted from its ordered position relative to a time-stamped packet. For example, if an initial packet is time-stamped with an initial real time, and one packet is stored every second, a 10^{th} packet following the initial packet can be associated with a real-time of 10 seconds following the initial real time. In some embodiments, a time stamp is inserted into any packet that includes an indicator of an external event signaled using event actuator **44** (Fig. **2-A**). In some embodiments, asynchronous events such as external event markers are marked by inserted flags, rather than explicit time-stamps. In some embodiments, time-stamps are inserted periodically in the packet sequence. In some embodiments, a time-stamp is inserted in a final packet any time recording is paused or ended, and in a first packet any time recording is started or resumed.

In some embodiments, packet assembly logic **126** or memory interface **134** may include encryption logic for encrypting physiologic monitoring data prior to storage in digital memory **32**. The encryption logic may implement a cipher such as a symmetric key cipher (e.g. RC4, AES), or a public-key cipher (e.g. RSA).

Audio/video output control logic **130** generates driving signals for light/sound indicator **42** (Fig. **2-A**), in response to trigger signals received from condition detection logic **124** and digital memory **32**, and in response to real-time data received from real-time clock **62**. In some embodiments, A/V output control logic **130** comprises audio decoding logic capable of decoding audio (e.g. speech) data stored in digital memory **32**. Speech data may be programmed in digital memory **32** during a system initialization, which may include steps such as setting the system real-time clock. In some embodiments, A/V output control logic **130** comprises logic configured to compare locally-stored time reference values to real-time data received from real-time clock **62**, and to generate driving signals when pre-set comparison conditions are met (e.g. every N minutes or hours, or at predetermined times/dates). A/V output control logic **130** sends decoded digital signals to a digital-to-analog converter (DAC), which may be provided as part of integrated circuit **30** or light/sound indicator **42**.

The driving signals generated by A/V output control logic **130** may include signals driving light/sound indicator **42** to flash an LED or change the LED display color, generate beeps, play music, or play digitized or synthesized voice prompts. Data used to generate the driving signals may include data hard-coded within integrated circuit **30** and/or data stored in digital memory **32.** In some embodiments, A/V output control logic **130** checks for a value of a digital speech flag in digital memory **32**. If the flag is set to an enabled value, A/V output control logic **130** retrieves the speech for transmission to light/sound indicator **42**. The digital speech flag is set during the system initialization.

Debounce and switch interface circuitry **132** receives external, analog event signals from event actuator **44** (Figs. **2-A-B**), and generates a clean digital event marker signal. The event marker signal is transmitted to packet assembly logic **126** for assembly into an associated packet. In some embodiments, debounce and switch interface circuitry **132** may be external to digital control logic **60**.

If integrated circuit **30** is configured in a stand-alone mode, the various parts of digital control logic **60** described above are active. If integrated circuit **30** is configured in a peripheral mode, at least some of the parts of digital control logic **60** are bypassed/disabled. In a self-clocked peripheral mode, packet assembly logic **126** generates a hardware interrupt to microcontroller **80** when an assembled data packet is ready. In the self-clocked peripheral mode, digital control logic **60** transfers data to microcontroller **80** for further processing and/or storage, rather than directly to digital memory **32**. Microcontroller **80** receives the assembled data packets, performs further processing on the data, and stores resulting data in a digital memory. In a passive peripheral mode, microcontroller **80** (Fig. **2-B**) perfonns the functions described above for digital control logic **60**, and digital control logic **60** is essentially disabled/bypassed. For example, in the passive peripheral mode, microcontroller **80** may provide synchronization timing signals to real-time clock **62**, supply digitized speech data to light/sound indicator **42**, control the timing of A/D converter **58**, control drive/signal processing circuit **52** to generate signals used for impedance measurements and fault detection.

In some embodiments, an initialization sequence as described above is performed according to the operating mode of integrated circuit **30.** In particular, if integrated circuit **30** is configured in a stand-alone mode, in some embodiments the initialization sequence may not include downloading firmware for microcontroller **80.** If integrated circuit **30** is configured in a passive peripheral mode, the initialization sequence may not include configuring digital control logic **60.** If integrated circuit **30** is configured in a self-clocked peripheral mode, the initialization sequence may include both downloading firmware for microcontroller **80** and programming a programmable logic array within digital control logic **60.**

In some embodiments, if integrated circuit **30** is configured in a peripheral mode, an initialization sequence as described above may be performed according to communications between microcontroller **80** and initialization console **260** (Fig. **6**), while initialization logic **122** (Fig. **4**) remains inactive. Microcontroller **80** may be connected to initialization console **260**, placed in a circuit program mode, and the entire operating firmware for microcontroller **80** may be downloaded to internal memory or digital memory **32**. Prior to initialization, microcontroller **80** may be blank, in order to make firmware piracy more difficult. After the initial programming, microcontroller **80** may allow changes to physiologic monitor configuration parameters such as enabled sensor set, sampling rates, real time, encryption key, authorization codes, subject/device IDs, voice/sound prompts, and other customization data stored in digital memory **32** or microcontroller **80**, while disallowing changes to the firmware.

In some embodiments, initialization/configuration sequences as described above may be used in physiologic monitors comprising a microcontroller and connected discrete components. Such initialization sequences may be used in physiologic monitors that do not include digital control logic and other components as described above.

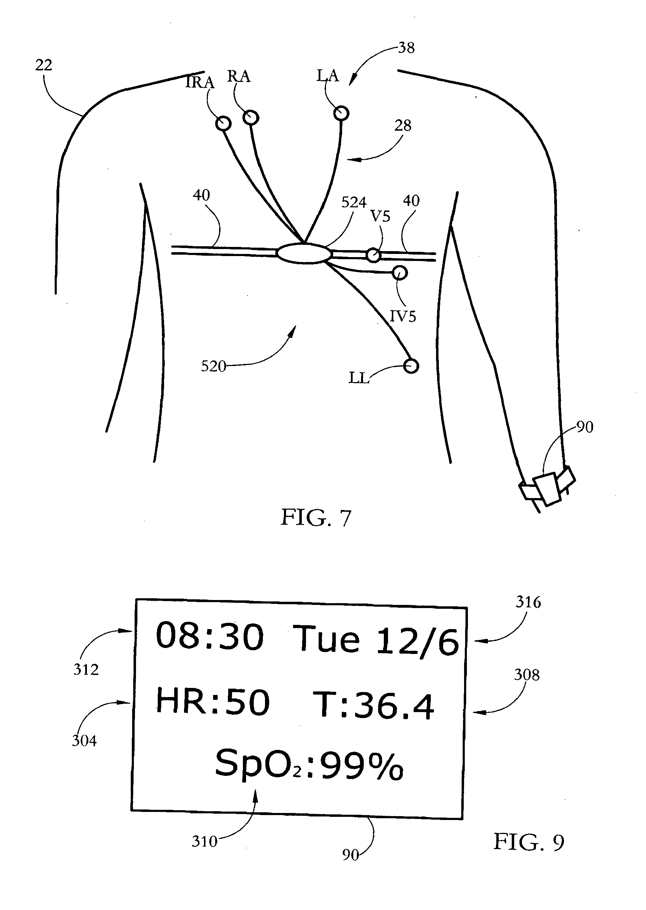

Fig. 7 shows a wearable physiologic monitor system **520** including a wearable display **90** connected to a wearable physiologic monitor **524** through a short-range wireless connection. Display **90** may be worn by a subject on his/her wrist, and may include or form part of a watch capable of displaying a real time. The short-range wireless connections may be implemented using a standard such as a Class 2 (2.5 mW) Bluetooth specification, allowing connections over a distance of 10 meters. Alternatively, a wired connection may be used to minimize cost or unwanted RF radiation.

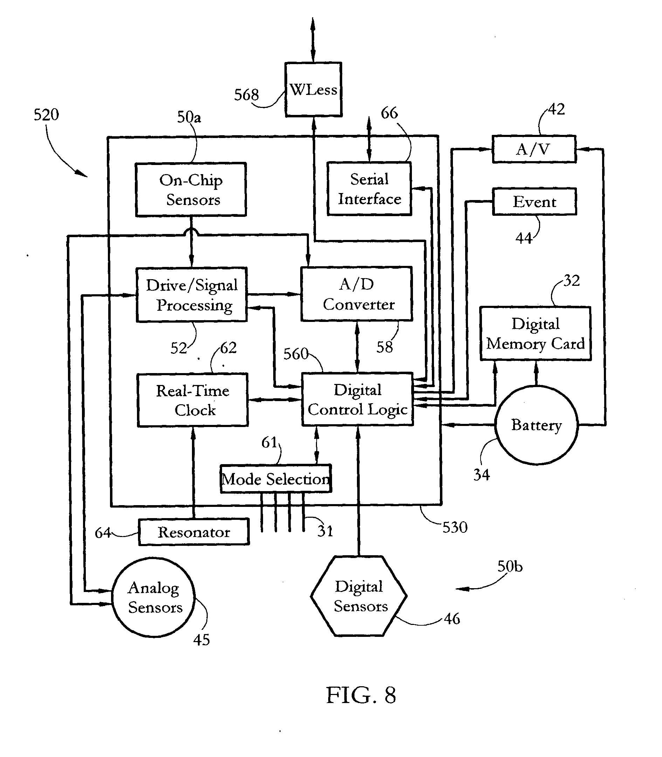

Fig. **8** is a diagram of physiologic monitor **524.** Physiologic monitor **524** includes an integrated circuit **530** connected to an off-chip wireless interface **568**. Wireless interface **568** may be connected to or include a wireless antenna, which is used to couple interface **568** to display 90. Wireless interface **568** is connected to digital control logic **560**. Digital control logic **560** may include wireless packet assembly logic assembling physiologic data packets for transmission to display **90**. The display data may include a subset of the sensor data types stored in digital memory **32**, and may be sent to wireless interface **568** at more frequent or less frequent time intervals than the data packets stored in digital memory **32**.

Fig. **9** shows an exemplary display **90** showing recorded real-time, time-dependent physiologic data. Display **90** presents to a user indications of several physiologic parameters determined by physiologic monitor **524** (Fig. **7**), including a heart rate display **304**, a current subject body temperature display **308**, and a current subject blood oxygen level **310**, among others. Display **90** also presents a real-time indicator **312** and a current date indicator **316**. In some embodiments, display **90** may receive and display a time-dependent signal waveform or trend, such as a current ECG waveform covering a few seconds, or a heart rate plot sampled once a minute over one hour.

According to one aspect, preferred systems and method described above allow producing a compact, low-cost, wearable multi-parameter physiologic monitor. Such a monitor may include a disposable patch encapsulating physiologic sensors, a processing integrated circuit, and sensor interconnections. Signal processing circuitry, a real-time clock, an A/D converter, an I/O port, a digital memory interface, and digital control logic are integrated on a single integrated circuit, which provides signal conditioning, time-stamping, multi-data-rate stream processing, and data packet assembly for storage in a removable digital memory such as a flash memory card. Using an application-specific integrated circuit (ASIC) including integrated special-purpose signal conditioning circuitry and digital control logic, rather than a programmable microcontroller and discrete front-end circuitry, allows producing a compact, power-efficient physiologic monitoring patch than can be readily made disposable. Storing the data in a removable digital memory also allows a simplified integrated circuit design, allowing device portability without requiring relatively-complex, power-consuming wireless transmission circuitry. The digital memory may be reused with multiple physiologic monitoring patches, while the wearable patch and its incorporated sensors and ASIC may be discarded at regular intervals, e.g. every 24 hours.

According to one aspect, the same physiologic monitor and incorporated integrated circuit design may be used in multiple-modes, each of which may be selected upon manufacture of the physiologic monitor. In a stand-alone mode, the physiologic monitor digital control logic performs time-stamping and packet assembly, and stores data to the digital memory. The stand-alone mode is particularly suited to applications in which portability, disposability, and low-cost are of primary concern. In a set of peripheral modes, the monitor digital control logic is used in conjunction with a microcontroller, which may be mounted on the subject or disposed nearby (e.g. in a bedside, larger unit). Using a programmable microcontroller allows greater flexibility in the data processing performed, but reduces the portability and increases the cost of the monitoring system. The peripheral modes are particularly suited to more stable environments, for example for in-hospital, bedside uses. In a passive peripheral mode, the physiologic monitor digital control logic is essentially bypassed, and the microcontroller performs operations such as control of an A/D converter, time-stamping, and packet assembly. In a self-clocked peripheral mode, the physiologic monitor digital control logic sends hardware interrupts to the microcontroller to periodically transfer time-stamped, assembled data packets to the microcontroller. Allowing a single integrated circuit design to be used in multiple modes allows sharing common design and fixed costs for both simpler standalone applications and more complex, bedside applications. For such bedside applications, the integrated circuit effectively acts as a signal processing front end for a programmable microprocessor such as a microcontroller.

According to another aspect, an initialization sequence, initialization logic and initialization console, as well as other aspects described above may be used in physiologic monitoring systems using discrete components for signal conditioning circuitry and digital control logic, rather than systems using an integrated circuit as described above. According to another aspect, such an initialization approach may be used to initialize a microcontroller rather than hardwired digital control logic, for example in a system configured in a peripheral mode, or in a system otherwise using a programmable microcontroller. In such a system, an initialization sequence may be implemented using initialization software running on the microcontroller. The authenticated firmware-download aspects described above are of particular significance in a system using a microcontroller. According to another aspect, such an initialization approach may be used in a system using a fixed digital memory. In some embodiments, such an initialization approach allows flexible customization and monetization of a common design for different uses in the field, for example in a doctor's office, and allows reducing device piracy.

According to another aspect, exemplary circuitry as described above may be used in a wearable physiologic monitoring system including a case or other structure loosely attached to a subject (e.g. worn inside clothing, or hanging from the subject), rather than a patch adhered to the subject.

According to anther aspect, the present invention provides computer-readable media encoding instructions, and computer systems programmed with instructions to perform the initialization and/or configuration steps described above, as well as other physiologic monitoring steps.

According to another aspect, a kit comprising a reusable, removable digital memory and a plurality of disposable, wearable physiologic monitors as described above is provided. For example, one digital memory may be provided together with 7 or 10 disposable physiologic monitors, each to be used by a subject for one day. The physiologic monitors provided in the kit are blank or otherwise user-inoperable to record subject physiologic data. The digital memory is pre-loaded with subject-specific physiologic monitor authorization and configuration data. The pre-loading may be performed for example in a physician's office, using an initialization console as described above, and according to subject-specific monitoring requirements. Connecting a blank physiologic monitor to the removable digital memory causes an activation and configuration of the physiologic monitor as described above. The activation results in the monitor becoming user-operable to record physiologic monitoring data. Subsequently, recorded physiologic monitoring data is stored in the removable digital memory.

According to some embodiments, a physiologic monitoring system as described above is employed in a long-term, patient-administered method of providing titration of care for a chronic disease, for which a compact, low-cost design as described above is of particular benefit. In an exemplary method, a physician issues one or more physiologic monitoring devices as described above to a patient in a first treatment state. The first treatment state may be a state in which the patient undergoes no treatment, or a state in which the patient undergoes a course of treatment, e.g. takes a particular medication. The patient uses the monitor(s) to record and store physiologic monitoring parameters over a period of days, weeks, or months, during a period of normal activity of the subject outside a medical facility. Such physiological monitoring may include detection of normal subject activity and physiologic parameters, as well as detection of less-common episodes such as syncope (fainting) or arrhythmias. Detected physiologic parameters may include the parameters described above, as well as parameters derived from measured data. For example, patient calorie expenditures due to physical activity may be estimated from recorded accelerometer data, while patient heart failure progression can be estimated using fluid load impedance measurements. Generating the data need not require the patient's return to a medical facility. Quantitative data generated over a period of time is used in conjunction with other data, including pre-treatment baseline data, to evaluate the effect of treatment on the patient, and to alter the course of therapy (e.g. medication dosages). New data for the altered course of therapy are then recorded. Quantitative data generated by different courses of treatment are then compared.

Quantitative data evaluation may include monitoring a time-dependence of relevant parameters as a course of treatment proceeds, comparing such measured parameters to pre-treatment baseline data, and evaluating effects of treatment changes on measured parameters. In some embodiments, quantitative data evaluation may include one or more of: comparing data during after/therapy to a pre-treatment baseline; comparing two or more medications or other treatments to each other; comparing two or more medications or other treatments to a pre-treatment baseline; comparing different dosages of one or more medications or other treatments, alone or in combination, to each other and/or to a pre-treatment baseline; comparing different combinations of medications or other treatments; evaluating non-medication treatment parameters, such as settings of implantable devices (e.g. pacemakers); evaluating implantable device settings in combination with one or more medications; comparing data recorded before and after surgical procedures. The preferred physiologic designs described above are particularly suited for such methods, and in particular for long-term (over weeks or months) tracking of patients' health by performing quantitative evaluations as described above.

Commonly available physiologic monitors such as Holter monitors are normally too complex and costly to permit convenient, widespread distribution of such monitors to patients for self-use outside of medical facilities. Qualitative evaluations by a patient (e.g. statements such as "I feel fine") are not particularly useful for evaluating subtle effects of medications or other treatment on the patient. A subject may go about his or her normal daily routines while data is recorded, and return to a physician's office at intervals spaced apart over a period of days or weeks to allow a physician to download stored physiologic data, perform a quantitative evaluation of the course of treatment according to the downloaded data, and adjust the course of treatment according to the evaluation.

According to another aspect, the basic physiologic monitor integrated circuit design supports multiple sensor types. The same physiologic monitor design may be used with multiple, different sensor subsets, as needed in a particular application, by enabling a desired sensor subset during the physiologic monitor manufacture or during an initialization of the physiologic monitor through an I/O (e.g. serial) port.

For more sophisticated data analysis, the digital memory may be removed from the physiologic monitor and connected to a personal computer or other device including a general-purpose CPU and associated analysis software. For example, a computer screen may be used to visualize data-intensive sensor displays such as time-dependent ECG traces.

In some embodiments, wireless transmission circuitry may be added to the physiologic monitor and connected to the data processing integrated circuit in order to provide a short-range connection to a wearable display unit. Adding wireless transmission circuitry to the physiologic monitor can add significant complexity, power losses, and noise to the system. Thus, a presently preferred implementation does not include wireless transmission circuitry.

Noise is of particular concern in systems measuring subject ECG signals, which may be on the order of 1 mV full-scale, and include frequencies of interest on the order of Hz. In addition to generally-present noise sources such as power lines, microwave and wireless sources, and electrolytic effects at the subject's skin, an integrated circuit designed as described above may also be affected by noise from an on-chip real-time clock tick signal or its harmonics. The effects of such noise on physiologic signal processing circuitry is preferably minimized by physically separating the real-time clock from the physiologic signal processing circuitry on opposite areas of a common substrate, and/or softening edges of a real-time tick signal, among others.

An integrated temperature sensor is also potentially subject to noise or extraneous inputs due to power losses from the integrated circuit itself, rather than heat emissions from the subject's skin. To minimize such noise sources, it is preferable to minimize the power losses from the integrated circuit, as well as optimize the thermal coupling between the temperature sensor and the subject.

It will be clear to one skilled in the art that the above embodiments may be altered in many ways. For example, a physiologic monitoring system as described above may include some asynchronous circuitry or logic. In some embodiments, various connections may be used to connect a physiologic monitor to an external computer or microcontroller, including wired (e.g. serial, USB, parallel) and wireless (optical, RF) connections. Accordingly, the scope of the invention should be determined by the following claims.

## Claims

1. A physiologic monitoring method comprising:
storing a set of physiologic monitor authorization and configuration data in a removable digital memory (32'), the authorization and configuration data including firmware;
transitioning a wearable physiologic monitor (24) from an un-initialized state to an initialized state using the physiologic monitor authorization and configuration data stored in the removable digital memory (32') wherein, if a user has been determined to be authorized, the initialization logic (122) of the physiologic monitor (24) retrieves the firmware for a microcontroller (80) of the wearable physiologic monitor (24) from the removable digital memory (32'), and wherein the wearable physiologic monitor (24) in the un-initialized state is not user-operable to record a set of physiologic monitoring data from the subject (22), and wherein the wearable physiologic monitor (24) in the initialized state is user-operable to record the set of physiologic monitoring data from the subject (22);
employing the wearable physiologic monitor (24) including the removable digital memory (32') to record the set of physiologic monitoring data on the subject (22) wearing the wearable physiologic monitor (24); and
storing the set of physiologic monitoring data in the removable digital memory (32').

2. The method of claim 1, further comprising:
transmitting the physiologic monitor authorization and/or configuration data including the firmware to the removable digital memory (32').

3. The method of claim 1, further comprising:
connecting the removable digital memory (32') to an initialization console (260) to store the set of physiologic monitor authorization and configuration data including the firmware for the wearable physiologic monitor (24) in the removable digital memory (32') while the wearable physiologic monitor (24) is not connected to the initialization console (260).

4. The method of any preceding claim,
wherein the set of physiologic monitor authorization and configuration data stored in the removable digital memory (32') is further adapted to configure a set of physiologic sensing operations performed by the physiologic monitor (24); and
wherein the set of physiologic monitoring data is recorded according to the configuration data.

5. The method of any preceding claim, further comprising storing in the removable digital memory (32') an encryption key to be used by the wearable physiologic monitor (24) to encrypt the physiologic monitoring data.

6. The method of any preceding claim, further comprising storing in the removable digital memory (32') an identifier of a payment transaction for the subject's use of the physiologic monitor.

7. The method of any preceding claim, wherein the set of physiologic monitoring data includes a set of electrocardiogram measurements.

8. The method of any preceding claim, wherein the set of physiologic monitoring data includes a set of 3-axis accelerometer measurements indicative of an orientation and activity of the subject.

9. The method of any preceding claim, further comprising storing a set of subject feedback data in the removable digital memory (32').

10. The method of any preceding claim, further comprising:
receiving a payment from a payor for a subject-specific set of authorized monitoring uses of the wearable physiologic monitor (24); and
activating the set of authorized monitoring uses of the wearable physiologic monitor (24) by programming the set of physiologic monitor authorization and configuration data in the removable digital memory (32') of the physiologic monitor (24).

11. A wearable physiologic monitoring system comprising:
a wearable physiologic monitor (24) comprising a microcontroller (80); and
a removable digital memory (32');
wherein the removable digital memory (32') is adapted to store a set of physiologic monitor authorization and configuration data, the physiologic monitor authorization and configuration data including firmware;
wherein the wearable physiologic monitor (24) is configured to be transitioned from an un-initialized state to an initialized state using the physiologic monitor authorization and configuration data stored in the removable digital memory (32'), wherein the initialization logic (122) of the physiologic monitor (24) is configured to, if a user has been determined to be authorized, retrieve the firmware for the microcontroller (80) of the wearable physiologic monitor (24) from the removable digital memory (32'), and wherein the wearable physiologic monitor (24) in the un-initialized state is not user-operable to record a set of physiologic monitoring data from the subject (22), and wherein the wearable physiologic monitor (24) in the initialized state is user-operable to record the set of physiologic monitoring data from the subject (22); and
wherein the wearable physiologic monitor (24) including the removable digital memory (32') is configured to record the set of physiologic monitoring data on the subject (22) wearing the wearable physiologic monitor (24) and to store the set of physiologic monitoring data in the removable digital memory (32').

12. The wearable physiologic monitoring system of claim 11, wherein the wearable physiologic monitor (24) further comprises a set of physiologic sensors (38, 50b), signal conditioning circuitry (52) connected to the sensors (38, 50b), and digital control logic (60) connected to the signal conditioning circuitry (52); wherein the removable digital memory (32') is connected to the digital control logic (60); and wherein the physiologic monitor authorization and configuration data is adapted to configure a set of physiologic sensing operations performed by the physiologic monitor (24), and subject physiologic monitoring data recorded by the physiologic monitor (24).

13. A physiologic monitoring kit comprising:
the wearable physiologic monitoring system (20) according to claim 11 or 12; and
a plurality of disposable, wearable physiologic monitors in an un-initialized state.

## Patentansprüche

1. Physiologisches Überwachungsverfahren, das umfasst:
Speichern eines Satzes von Autorisierungs- und Konfigurationsdaten einer physiologischen Überwachungseinrichtung in einem entnehmbaren digitalen Speicher (32'), wobei die Autorisierungs- und Konfigurationsdaten eine Firmware umfassen;
Überführen einer tragbaren physiologischen Überwachungseinrichtung (24) von einem nicht initialisierten Zustand in einen initialisierten Zustand unter Verwendung der Autorisierungs- und Konfigurationsdaten der physiologischen Überwachungseinrichtung, die im entnehmbaren digitalen Speicher (32') gespeichert sind, wobei, wenn ein Benutzer als autorisiert bestimmt wurde, die Initialisierungslogik (122) der physiologischen Überwachungseinrichtung (24) die Firmware für einen Mikrocontroller (80) der tragbaren physiologischen Überwachungseinrichtung (24) vom entnehmbaren digitalen Speicher (32') abruft, und wobei die tragbare physiologische Überwachungseinrichtung (24) im nicht initialisierten Zustand nicht vom Benutzer betreibbar ist, um einen Satz von physiologischen Überwachungsdaten von der Person (22) aufzuzeichnen, und wobei die tragbare physiologische Überwachungseinrichtung (24) im initialisierten Zustand vom Benutzer betreibbar ist, um den Satz von physiologischen Überwachungsdaten von der Person (22) aufzuzeichnen;
Verwenden der tragbaren physiologischen Überwachungseinrichtung (24) mit dem entnehmbaren digitalen Speicher (32'), um den Satz von physiologischen Überwachungsdaten von der Person (22), die die tragbare physiologische Überwachungseinrichtung (24) trägt, aufzuzeichnen; und
Speichern des Satzes von physiologischen Überwachungsdaten im entnehmbaren digitalen Speicher (32').

2. Verfahren nach Anspruch 1, das ferner umfasst:
Übertragen der Autorisierungs- und/oder Konfigurationsdaten, der physiologischen Überwachungseinrichtung, einschließlich der Firmware, zum entnehmbaren digitalen Speicher (32').

3. Verfahren nach Anspruch 1, das ferner umfasst:
Verbinden des entnehmbaren digitalen Speichers (32') mit einer Initialisierungskonsole (260), um den Satz von Autorisierungs- und Konfigurationsdaten der physiologischen Überwachungseinrichtung, einschließlich der Firmware für die tragbare physiologische Überwachungseinrichtung (24), im entnehmbaren digitalen Speicher (32') zu speichern, während die tragbare physiologische Überwachungseinrichtung (24) nicht mit der Initialisierungskonsole (260) verbunden ist.

4. Verfahren nach einem vorangehenden Anspruch,
wobei der Satz von Autorisierungs- und Konfigurationsdaten der physiologischen Überwachungseinrichtung, die im entnehmbaren digitalen Speicher (32') gespeichert ist, ferner dazu ausgelegt ist, einen Satz von physiologischen Erfassungsoperationen zu konfigurieren, die durch die physiologische Überwachungseinrichtung (24) durchgeführt werden; und
wobei der Satz von physiologischen Überwachungsdaten gemäß den Konfigurationsdaten aufgezeichnet wird.

5. Verfahren nach einem vorangehenden Anspruch, das ferner das Speichern eines Verschlüsselungsschlüssels im entnehmbaren digitalen Speicher (32') umfasst, der von der tragbaren physiologischen Überwachungseinrichtung (24) verwendet werden soll, um die physiologischen Überwachungsdaten zu verschlüsseln.

6. Verfahren nach einem vorangehenden Anspruch, das ferner das Speichern eines Identifikators einer Bezahlungstransaktion im entnehmbaren digitalen Speicher (32') für die Verwendung der physiologischen Überwachungseinrichtung durch die Person umfasst.

7. Verfahren nach einem vorangehenden Anspruch, wobei der Satz von physiologischen Überwachungsdaten einen Satz von Elektrokardiogrammmessungen umfasst.

8. Verfahren nach einem vorangehenden Anspruch, wobei der Satz von physiologischen Überwachungsdaten einen Satz von 3-Achsen-Beschleunigungsmesser-Messungen umfasst, die eine Orientierung und Aktivität der Person angeben.

9. Verfahren nach einem vorangehenden Anspruch, das ferner das Speichern eines Satzes von Rückmeldungsdaten der Person im entnehmbaren digitalen Speicher (32') umfasst.

10. Verfahren nach einem vorangehenden Anspruch, das ferner umfasst:
Empfangen einer Bezahlung von einem Zahlenden für einen für die Person spezifischen Satz von autorisierten Überwachungsverwendungen der tragbaren physiologischen Überwachungseinrichtung (24); und
Aktivieren des Satzes von autorisierten Überwachungsverwendungen der tragbaren physiologischen Überwachungseinrichtung (24) durch Programmieren des Satzes von Autorisierungs- und Konfigurationsdaten der physiologischen Überwachungseinrichtung im entnehmbaren digitalen Speicher (32') der physiologischen Überwachungseinrichtung (24).

11. Tragbares physiologisches Überwachungssystem, das umfasst:
eine tragbare physiologische Überwachungseinrichtung (24) mit einem Mikrocontroller (80); und
einen entnehmbaren digitalen Speicher (32');
wobei der entnehmbare digitale Speicher (32') dazu ausgelegt ist, einen Satz von Autorisierungs- und Konfigurationsdaten der physiologischen Überwachungseinrichtung zu speichern, wobei die Autorisierungs- und Konfigurationsdaten der physiologischen Überwachungseinrichtung eine Firmware umfassen;
wobei die tragbare physiologische Überwachungseinrichtung (24) so konfiguriert ist, dass sie von einem nicht initialisierten Zustand in einen initialisierten Zustand unter Verwendung der Autorisierungs- und Konfigurationsdaten der physiologischen Überwachungseinrichtung, die im entnehmbaren digitalen Speicher (32') gespeichert sind, überführt wird, wobei die Initialisierungslogik (122) der physiologischen Überwachungseinrichtung (24) dazu ausgelegt ist, wenn ein Benutzer als autorisiert bestimmt wurde, die Firmware für den Mikrocontroller (80) der tragbaren physiologischen Überwachungseinrichtung (24) vom entnehmbaren digitalen Speicher (32') abzurufen, und wobei die tragbare physiologische Überwachungseinrichtung (24) im nicht initialisierten Zustand nicht vom Benutzer betreibbar ist, um einen Satz von physiologischen Überwachungsdaten von der Person (22) aufzuzeichnen, und wobei die tragbare physiologische Überwachungseinrichtung (24) im initialisierten Zustand vom Benutzer betreibbar ist, um den Satz von physiologischen Überwachungsdaten von der Person (22) aufzuzeichnen; und
wobei die tragbare physiologische Überwachungseinrichtung (24) mit dem entnehmbaren digitalen Speicher (32') konfiguriert ist, den Satz von physiologischen Überwachungsdaten über die Person (22), die die tragbare physiologische Überwachungseinrichtung (24) trägt, aufzuzeichnen und den Satz von physiologischen Überwachungsdaten im entnehmbaren digitalen Speicher (32') zu speichern.

12. Tragbares physiologisches Überwachungssystem nach Anspruch 11, wobei die tragbare physiologische Überwachungseinrichtung (24) ferner einen Satz von physiologischen Sensoren (38, 50b), eine Signalkonditionierungs-Schaltungsanordnung (52), die mit den Sensoren (38, 50b) verbunden ist, und eine digitale Steuerlogik (60), die mit der Signalkonditionierungs-Schaltungsanordnung (52) verbunden ist, umfasst; wobei der entnehmbare digitale Speicher (32') mit der digitalen Steuerlogik (60) verbunden ist; und wobei die Autorisierungs- und Konfigurationsdaten der physiologischen Überwachungseinrichtung dazu ausgelegt sind, einen Satz von physiologischen Erfassungsoperationen, die von der physiologischen Überwachungseinrichtung (24) durchgeführt werden, und physiologische Überwachungsdaten der Person, die durch die physiologische Überwachungseinrichtung (24) aufgezeichnet werden, zu konfigurieren.

13. Ausrüstung zur physiologischen Überwachung, die umfasst:
das tragbare physiologische Überwachungssystem (20) nach Anspruch 11 oder 12; und
eine Vielzahl von wegwerfbaren, tragbaren physiologischen Überwachungseinrichtungen in einem nicht initialisierten Zustand.

## Revendications

1. Procédé de surveillance physiologique comprenant les étapes consistant à :
mémoriser un ensemble de données d'autorisation et de configuration de dispositif de surveillance physiologique dans une mémoire numérique amovible (32'), les données d'autorisation et de configuration incluant un microprogramme,
faire effectuer une transition à un dispositif de surveillance physiologique pouvant être porté (24) d'un état non initialisé à un état initialisé en utilisant les données d'autorisation et de configuration de dispositif de surveillance physiologique mémorisées dans la mémoire numérique amovible (32') dans lequel, si un utilisateur a été déterminé comme étant autorisé, la logique d'initialisation (122) du dispositif de surveillance physiologique (24) récupère le microprogramme pour un microcontrôleur (80) du dispositif de surveillance physiologique pouvant être porté (24) à partir de la mémoire numérique amovible (32'), et dans lequel le dispositif de surveillance physiologique pouvant être porté (24) dans l'état non initialisé ne peut pas être utilisé par un utilisateur pour enregistrer un ensemble de données de surveillance physiologique à partir du sujet (22), et dans lequel le dispositif de surveillance physiologique pouvant être porté (24) dans l'état initialisé peut être utilisé par un utilisateur pour enregistrer l'ensemble de données de surveillance physiologique à partir du sujet (22),
employer le dispositif de surveillance physiologique pouvant être porté (24) incluant la mémoire numérique amovible (32') pour enregistrer l'ensemble de données de surveillance physiologique sur le sujet (22) portant le dispositif de surveillance physiologique pouvant être porté (24), et
mémoriser l'ensemble de données de surveillance physiologique dans la mémoire numérique amovible (32').

2. Procédé selon la revendication 1, comprenant en outre l'étape consistant à :
transmettre les données d'autorisation et/ou de configuration de dispositif de surveillance physiologique incluant le microprogramme à la mémoire numérique amovible (32').

3. Procédé selon la revendication 1, comprenant en outre l'étape consistant à :
connecter la mémoire numérique amovible (32') à une console d'initialisation (260) pour mémoriser l'ensemble de données d'autorisation et de configuration de dispositif de surveillance physiologique incluant le microprogramme pour le dispositif de surveillance physiologique pouvant être porté (24) dans la mémoire numérique amovible (32') alors que le dispositif de surveillance physiologique pouvant être porté (24) n'est pas connecté à la console d'initialisation (260).

4. Procédé selon l'une quelconque des revendications précédentes,
dans lequel l'ensemble de données d'autorisation et de configuration de dispositif de surveillance physiologique mémorisé dans la mémoire numérique amovible (32') est en outre destiné à configurer un ensemble d'opérations de détection physiologique exécutées par le dispositif de surveillance physiologique (24), et
dans lequel l'ensemble de données de surveillance physiologique est enregistré selon les données de configuration.

5. Procédé selon l'une quelconque des revendications précédentes, comprenant en outre la mémorisation dans la mémoire numérique amovible (32') d'une clé de cryptage devant être utilisée par le dispositif de surveillance physiologique pouvant être porté (24) pour crypter les données de surveillance physiologique.

6. Procédé selon l'une quelconque des revendications précédentes, comprenant en outre la mémorisation dans la mémoire numérique amovible (32') d'un identificateur d'une transaction de paiement pour l'utilisation par le sujet du dispositif de surveillance physiologique.

7. Procédé selon l'une quelconque des revendications précédentes, dans lequel l'ensemble de données de surveillance physiologique inclut un ensemble de mesures d'électrocardiogramme.

8. Procédé selon l'une quelconque des revendications précédentes, dans lequel l'ensemble de données de surveillance physiologique inclut un ensemble de mesures d'accéléromètre à 3 axes indicatives d'une orientation et d'une activité du sujet.

9. Procédé selon l'une quelconque des revendications précédentes, comprenant en outre la mémorisation d'un ensemble de données de retour de sujet dans la mémoire numérique amovible (32').

10. Procédé selon l'une quelconque des revendications précédentes, comprenant en outre les étapes consistant à :
recevoir un paiement d'un payeur pour un ensemble spécifique à un sujet d'utilisations de surveillance autorisées du dispositif de surveillance physiologique pouvant être porté (24), et
activer l'ensemble d'utilisations de surveillance autorisées du dispositif de surveillance physiologique pouvant être porté (24) en programmant l'ensemble de données d'autorisation et de configuration de dispositif de surveillance physiologique dans la mémoire numérique amovible (32') du dispositif de surveillance physiologique (24).

11. Système de surveillance physiologique pouvant être porté comprenant :
un dispositif de surveillance physiologique pouvant être porté (24) comprenant un microcontrôleur (80), et
une mémoire numérique amovible (32'),
dans lequel la mémoire numérique amovible (32') est destinée à mémoriser un ensemble de données d'autorisation et de configuration de dispositif de surveillance physiologique, les données d'autorisation et de configuration de dispositif de surveillance physiologique incluant un microprogramme,
dans lequel le dispositif de surveillance physiologique pouvant être porté (24) est configuré pour effectuer une transition d'un état non initialisé à un état initialisé en utilisant les données d'autorisation et de configuration de dispositif de surveillance physiologique mémorisées dans la mémoire numérique amovible (32'), récupère le microprogramme pour le microcontrôleur (80) du dispositif de surveillance physiologique pouvant être porté (24) à partir de la mémoire numérique amovible (32'), et
dans lequel le dispositif de surveillance physiologique pouvant être porté (24) dans l'état non initialisé ne peut pas être utilisé par un utilisateur pour enregistrer un ensemble de données de surveillance physiologique à partir du sujet (22), et dans lequel le dispositif de surveillance physiologique pouvant être porté (24) dans l'état initialisé peut être utilisé par un utilisateur pour enregistrer l'ensemble de données de surveillance physiologique à partir du sujet (22), et
dans lequel le dispositif de surveillance physiologique pouvant être porté (34) incluant la mémoire numérique amovible (32') est configuré pour enregistrer l'ensemble de données de surveillance physiologique sur le sujet (22) portant le dispositif de surveillance physiologique pouvant être porté (24) et pour mémoriser l'ensemble de données de surveillance physiologique dans la mémoire numérique amovible (32').

12. Système de surveillance physiologique pouvant être porté selon la revendication 11, dans lequel le dispositif de surveillance physiologique pouvant être porté (24) comprend en outre un ensemble de capteurs physiologiques (38, 50b), des circuits de conditionnement de signaux (52) connectés aux capteurs (38, 50b), et une logique de commande numérique (60) connectée aux circuits de conditionnement de signaux (52), dans lequel la mémoire numérique amovible (32') est connectée à la logique de commande numérique (60), et dans lequel les données d'autorisation et de configuration de dispositif de surveillance physiologique sont destinées à configurer un ensemble d'opérations de détection physiologique exécutées par le dispositif de surveillance physiologique (24), et les données de surveillance physiologique de sujet enregistrées par le dispositif de surveillance physiologique (24).

13. Ensemble de surveillance physiologique comprenant :
le système de surveillance physiologique pouvant être porté (20) selon la revendication 11 ou 12, et
une pluralité de dispositifs de surveillance physiologique pouvant être portés amovibles dans un état non initialisé.
